# EUROPEAN PATENT SPECIFICATION

(11) **EP 1 292 586 B1**
(45) Date of publication and mention of the grant of the patent: **04.08.2004**
(21) Application number: 01936737.4
(22) Date of filing: 11.06.2001
(51) Int. Cl.: C07D 295/22, A61P 15/00, A61K 31/495

(54) **PROCESS FOR THE PREPARATION OF PYRAZOLOPYRIMIDINONES**
VERFAHREN ZUR HERSTELLUNG VON PYRAZOLOPYRIMIDINONEN
PROCEDE DE PREPARATION DE PYRAZOLOPYRIMIDINONES

(30) Priority: 22.06.2000 GB 0015472; 09.03.2001 GB 0105857
(43) Date of publication of application: 19.03.2003
(73) Proprietor: Pfizer Limited, Sandwich, Kent CT13 9NJ (GB); PFIZER INC., New York, N.Y. 10017 (US)
(72) Inventor: DUNN, Peter, James, Pfizer Global Res. & Dev., Sandwich, Kent CT13 9NJ (GB); DUNNE, Catherine, Pfizer Global Res. & Dev., Sandwich, Kent CT13 9NJ (GB)
(74) Representative: Atkinson, Jonathan David Mark
(86) International application number: PCT/IB2001/001050
(87) International publication number: WO 2001/098284

(56) References cited:
- EP-A- 0 994 115
- EP-A- 1 002 798
- D. P. ROTELLA ET AL.: "N-3-Substituted Imidazoquinolinones: Potent and Selective PDE5 Inhibitors as Potential Agents for Treatment od Erectile Dysfunction" J. MED. CHEM., vol. 43, no. 7, April 2000 (2000-04), pages 1257-1263, XP002177561
- COSTANZO M J ET AL: "POTENT THROMBIN INHIBITORS THAT PROBE THE S1' SUBSITE: TRIPEPTIDE TRANSITION STATE ANALOGUES BASED ON A HETEROCYCLE-ACTIVATED CARBONYL GROUP" JOURNAL OF MEDICINAL CHEMISTRY, AMERICAN CHEMICAL SOCIETY. WASHINGTON, US, vol. 39, no. 16, 2 August 1996 (1996-08-02), pages 3039-3043, XP002024136 ISSN: 0022-2623
- PARKIN A ET AL: "ACYCLIC ANALOGUES OF PURINE AND IMIDAZOLE NUCLEOSIDES" JOURNAL OF HETEROCYCLIC CHEMISTRY, HETEROCORPORATION. PROVO, US, vol. 19, no. 33, January 1982 (1982-01), pages 33-40, XP001007465 ISSN: 0022-152X
- SCHNELLER S W ET AL: "SYNTHESIS OF PROXIMAL-BENZOGUANINE AND A SIMPLIFIED SYNTHESIS OF PROXIMAL-BENZOHYPOXANTHINE" JOURNAL OF ORGANIC CHEMISTRY, AMERICAN CHEMICAL SOCIETY. EASTON, US, vol. 51, no. 21, 1986, pages 4067-4070, XP002920282 ISSN: 0022-3263

## Description

This invention relates to a novel process for the production of 4-alkylpiperazinylsulfonylphenyl- and 4-alkylpiperazinylsulfonyl pyridinyldihydropyrazolo[4,3-d]pyrimidin-7-one derivatives, and, in particular, the anti-impotence drug, sildenafil and analogues thereof.

Sildenafil (5-[2-ethoxy-5-(4-methylpiperazin-1-ylsulfonyl)phenyl)-1-methyl-3-*n* propyl-1,6-dihydro-7*H* pyrazolo[4,3-d]pyrimidin-7-one), 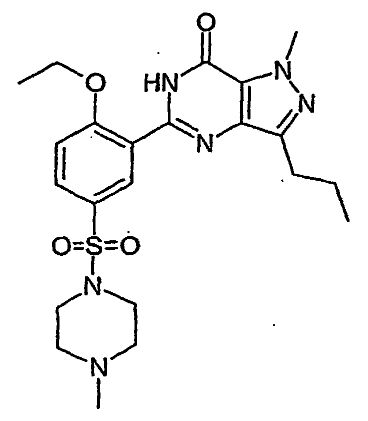 is the active ingredient in Viagra™. The compound, which was originally disclosed in European patent application EP 463 756, has been found to be particularly useful in the treatment of *inter alia* male erectile dysfunction (see international patent application WO 94/28902).

J. Heterocyclic Chem 1982, 19, 33, discloses the synthesis and antiviral activity of acyclic analogues of purine and imidazole nucleosides. A number of synthetic procedures, including cyclisation of an imidazole precursor, are disclosed.

Multi-step syntheses for the preparation of sildenafil are described in EP 463 756. An improved process for its production is described in a later application (European patent application EP 812 845), the final step of which involves an internal cyclisation under basic, neutral or acidic conditions.

We have now found that sildenafil and analogues thereof may be made via a novel process, as described hereinafter, which process has advantages over the processes described in the above-mentioned prior art documents.

According to a first aspect of the invention, there is provided a process for the production of compounds of general formula I: 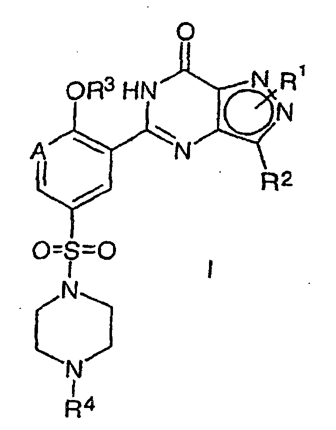 wherein
A represents CH or N;
R¹ represents H, C₁₋₆ alkyl (which alkyl group is optionally interrupted by O), Het, alkylHet, aryl or alkylaryl, which latter five groups are all optionally substituted (and/or, in the case of C₁₋₆ alkyl, optionally terminated) by one or more substituents selected from halo, cyano, nitro, lower alkyl, OR⁵, C(O)R⁶, C(O)OR⁷, C(O)NR⁸R⁹, NR^{10a}R^{10b} and SO₂NR^{11a}R^{11b};
R² and R⁴ independently represent C₁₋₆ alkyl;
R³ represents lower alkyl, which alkyl group is optionally interrupted by oxygen;
Het represents an optionally substituted four- to twelve-membered heterocyclic group, which group contains one or more heteroatoms selected from nitrogen, oxygen and sulfur; wherein Het groups containing N or S atoms may also be present in their N- or S- oxidised form,
R⁵, R⁶, R⁷, R⁸, R⁹, R^{11a} and R^{11b} independently represent H or lower alkyl;
R^{10a} and R^{10b} either independently represent, H or lower alkyl or, together with the nitrogen atom to which they are attached, represent azetidinyl, pyrollidinyl or piperidinyl, wherein lower alkyl means C₁₋₆ alkyl which when there is a sufficient number of carbon atoms, may be linear or branched, be saturated or unsaturated, be cyclic, acyclic or part cyclic/acyclic, and/or be substituted by one or more halo atoms,
which process comprises the reaction of a compound of formula II, 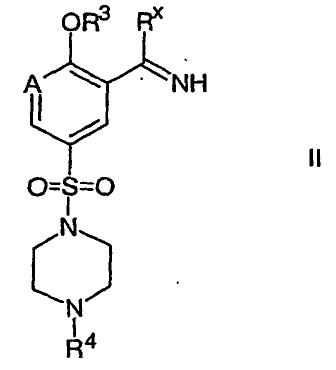 wherein R^{x} is a group substitutable by an aminopyrazole and A, R³ and R⁴ are as defined above,
with a compound of general formula III, 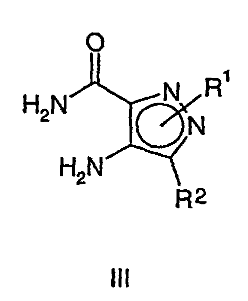 wherein R¹ and R² are as defined above,
which process is referred to hereinafter as "the process of the invention".

The compounds of the general formulae I and III may be represented by either of the formulae I, IA and IB or IHA or IIIB in the process according to the present invention.

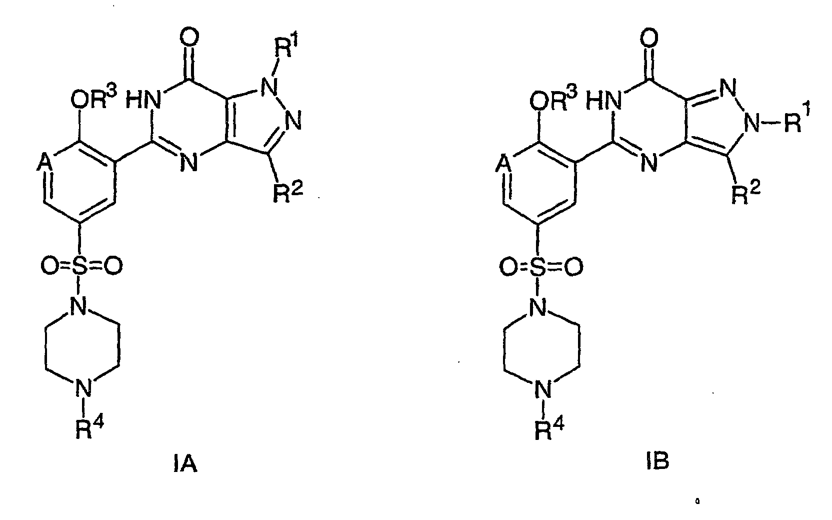

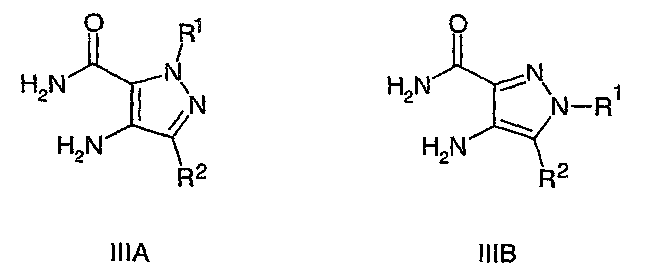

The term "aryl", when used herein, includes six- to ten-membered carbocyclic aromatic groups, such as phenyl and naphthyl and the like.

Het groups may be fully saturated, partly unsaturated, wholly aromatic, partly aromatic and/or bicyclic in character. Het groups that may be mentioned include groups such as optionally substituted azetidinyl, pyrrolidinyl, imidazolyl, indolyl, oxadiazolyl, thiadiazolyl, triazolyl, tetrazolyl, oxatriazolyl, thiatriazolyl, pyridazinyl, morpholinyl, pyrimidinyl, pyrazinyl, pyridyl, quinolinyl, isoquinolinyl, piperidinyl, pyrazolyl, imidazopyridinyl, piperazinyl, thienyl and furanyl.

The point of attachment of any Het group may be *via* any atom in the ring system including (where appropriate) a heteroatom. Het groups may also be present in the *N*- or *S*-oxidised form.

The term "lower alkyl" (which includes the alkyl part of alkylHet and alkylaryl groups), when used herein, includes C₁₋₆ alkyl (e.g. C₁₋₄ alkyl). Unless otherwise specified, alkyl groups may, when there is a sufficient number of carbon atoms, be linear or branched, be saturated or unsaturated, be cyclic, acyclic or part cyclic/acyclic, and/or be substituted by one or more halo atoms.

As defined herein, the term "halo" includes fluoro, chloro, bromo and iodo.

Compounds of formulae I, IA and IB may contain one or more asymmetric carbon atoms and may therefore exhibit optical and/or diastereoisomerism. The process of the invention thus also relates to the formation of stereoisomers of compounds of formulae I, IA and IB and mixtures thereof. Stereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation, or by derivatisation, for example with a homochiral acid followed by separation of the diastereomeric esters by conventional means (e.g. HPLC, crystallisation, chromatography over silica or, for example, *via* classical resolution with a homochiral acid salt). The formation of all stereoisomers is included within the scope of the invention.

Compounds of formula II may exhibit tautomerism. The use of all tautomeric forms of the compounds of formula II is included within the scope of the invention.

Preferred compounds of formulae I, IA and IB include those in which:
R¹ represents C₁₋₄, alkyl, which alkyl group is optionally interrupted by an oxygen atom, and/or is optionally terminated by a Het group (such as a pyridinyl group);
R² represents C₁₋₄ alkyl;
R³ represents C₁₋₅ alkyl, which alkyl group is optionally interrupted by an oxygen atom;
R⁴ represents C₁₋₃ alkyl.

More preferred compounds of formulae I, IA and IB include those in which:
R¹ represents linear C₁₋₃ alkyl, which alkyl group is optionally interrupted by an oxygen atom, or is optionally terminated by a 2-pyridinyl group (e.g. to form a 2-pyridinylmethyl group);
R² represents linear C₂₋₃ alkyl;
R³ represents linear or branched C₂₋₄ alkyl, which alkyl group is optionally interrupted by an oxygen atom;
R⁴ represents C₁₋₂ alkyl.

Particularly preferred compounds that may be formed in the process of the invention include sildenafil, and the following four compounds:

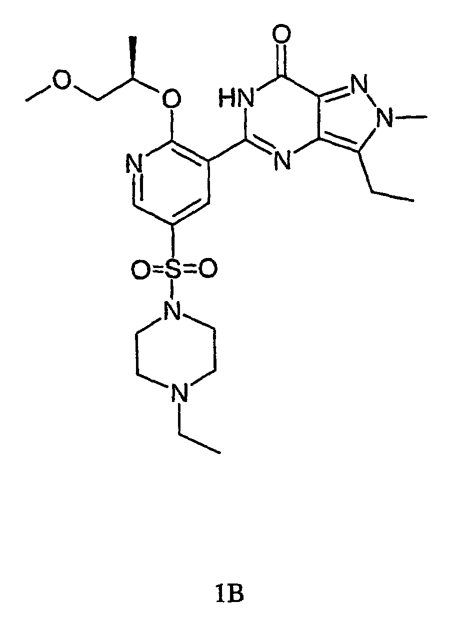

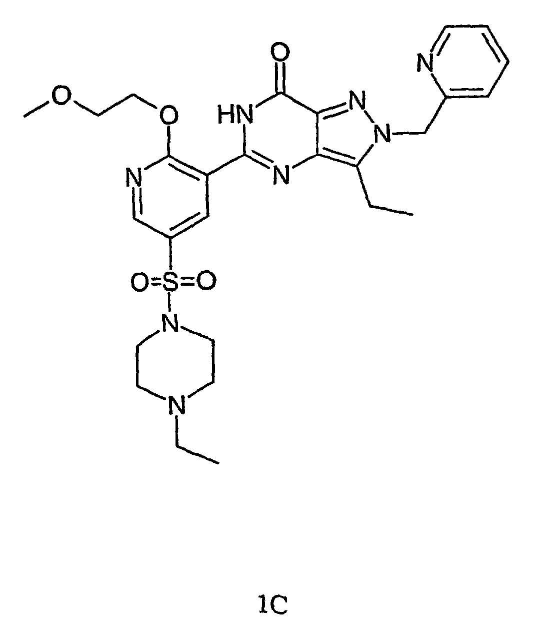

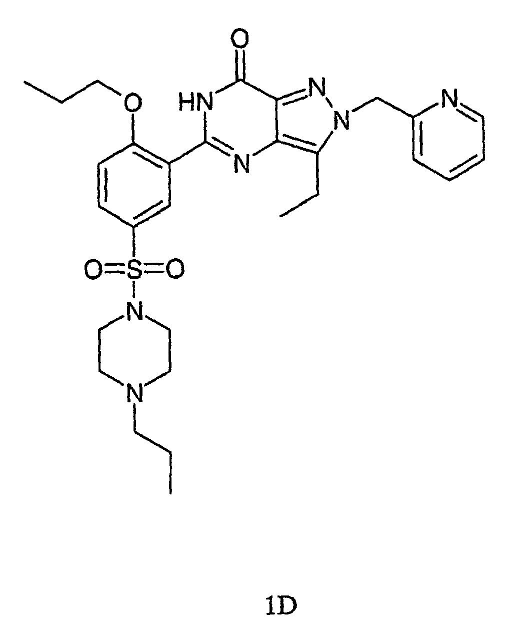

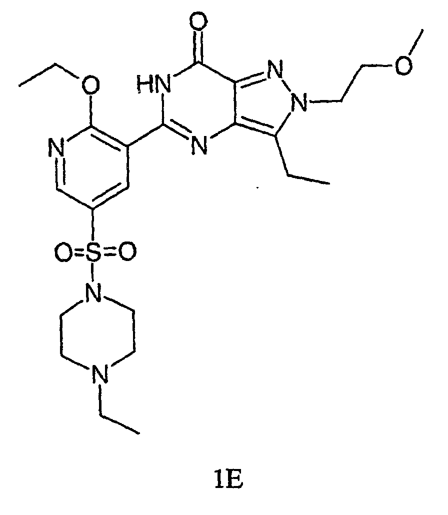

Said compounds 1B, 1C, 1D and 1E are otherwise known as: 1B, (+)-3-ethyl-5- [5-(4-ethylpiperazin-1 -ylsulphonyl)-2-(2-methoxy-1 (R)-methylethoxy)pyridin-3-yl]-2-methyl-2,6-dihydro-7H-pyrazolo[4,3-d]pyrimidin-7-one also known as 3-Ethyl-5-{5-[4-ethylpiperazin-1-ylsulphonyl]-2-([(1R)-2-methoxy-1-methylethyl)oxy)pyridin-3-yl}-2-methyl-2,6-dihydro-7H-pyrazolo[4,3-d] pyrimidin-7-one, the compound of Example 118 of WO99/54333 1C, 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one, the compound of Example 5 of WO98/49166; 1D, 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d)pyrimidin-7-one, the compound of Example 4 of WO99/54333; and 1E, 5-[2-Ethoxy-5-(4-ethylpiperazin-1-ylsulphonyl)pyridin-3-yl]-3-ethyl-2-[2-methoxyethyl)-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2*H* pyrazalo[4,3-d]pyrimidin-5-yl]-3-pyridyl sulphonyl}-4-ethylpiperazine, the compound of Example 8 of IB00/01457, exemplified hereinafter as Example 3.

By "group substitutable by an aminopyrazole having structure III" we include any group which, when present in the moiety -C(R^{x})=NH of a compound of formula II, may undergo displacement by the amino group of an aminopyrazole such that a -C(=NH)-NH- linkage is thereby formed. In accordance with the process of the invention, which the skilled person will appreciate involves a "one-pot" condensation/cyclisation reaction, the aminopyrazole that is reacted with the compound of formula II is a compound of formula III, IIIA or IIIB. Following the condensation reaction, the coupled intermediate undergoes cyclisation to form a compound of general formula I.

In this respect, preferred groups that R^{x} may represent include -NH₂, -NHR^{a}, -N(R^{b})R^{c}, -SR^{d}, -SH, -OR^{e} wherein groups R^{b} to R^{e} each independently represent the same groups that R¹ as hereinbefore defined may represent (except that they do not represent H or alkoxy) as well as halo (e.g. chloro). Group R^{a} represents -OR¹ or halo (e.g. chloro) wherein R¹ is as hereinbefore defined. More preferred values of R^{x} include -NHR^{a}, -N(R^{b})R^{c}, and preferably -SR^{d}, -SH and -OR^{e}. Particularly preferred values of R^{x} are C₁₋₄ alkoxy (e.g. ethoxy).

According to a further aspect of the invention, there is provided a process for the production of a compound of formula I, IA or IB, as hereinbefore defined, which process comprises the reaction of a compound of formula III, IIIA or IIIB (as appropriate), as hereinbefore defined, with a compound of formula II, as hereinbefore defined, provided that R^{x} does not represent -NH₂, or, preferably, R^{x} does not represent -NH₂, -NHR^{a} or -N(R^{b})R^{c}.

The process of the invention may be carried out in the presence of a suitable organic solvent system, which solvent system should not significantly react chemically with, or significantly give rise to stereochemical changes in, the reactants or product once formed, or significantly give rise to other side reactions. Preferred solvent systems include aromatic hydrocarbons (e.g. toluene or xylene) or chlorobenzene. Preferred solvent systems also include solvents of formula R^{x}H, for example, solvents of formula R^{e}OH (e.g. ethanol), wherein R^{x} and R^{e} are as hereinbefore defined.

In the process of the invention, it may be preferable to add compounds of formulae III, IIIA or IIIB to the reaction mixture (prior to carrying out the reaction) in a suitable polar organic solvent such as ethyl acetate. Such a polar solvent may then be removed before the reaction is initiated.

The process of the invention may be carried at elevated temperature (e.g. up to the reflux temperature of the relevant solvent system, or higher if a pressurised system is employed). Clearly, appropriate reaction times and reaction temperatures depend upon the solvent system that is employed, as well as the reactants that are used and the compound that is to be formed, but these may be determined routinely by the skilled person.

We have found that compounds of formula IT may be prepared, advantageously, by way of reaction of a compound of formula IV, 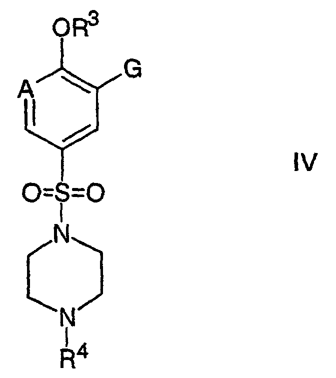 wherein G represents a carboxylic acid group (-C(O)OH) or a derivative thereof, and A, R³ and R⁴ are as hereinbefore defined, with an appropriate reagent for converting the group G to a -C(R^{x})=NH group, wherein R^{x} is as hereinbefore defined.

The term "derivative of a carboxylic acid group" when used herein includes groups which are commonly derived from a carboxylic acid and/or groups that contain a central carbon atom (which carbon atom is attached to the phenyl or pyridyl ring in the compound of formula IV) that is at the same oxidation state as -C(O)OH. The term therefore includes groups such as -CN, -C(OR^{e})₃, -C(O)NH₂ or -C(=NOR^{f})N(R^{e})₂, wherein R^{f} represents H or lower alkyl and R^{e} is as hereinbefore defined. G can also represent a 5- or 6-memebered heterocyclic group containing at least two heteroatoms selected from O, S, N and mixtures thereof wherein said heterocyclic group is bonded by a carbon atom, a preferred heterocyclic group, as exemplified in preparation 4 herein, has the general formula -C(=NOR^{g})N(R^{e}) wherein the carbon is bonded to both N atoms and wherein R^{g} is bonded to the N of the -NR^{e} group and wherein R^{g} is a -CH, or a -CH₂- group and wherein R^{e} is as defined hereinbefore and is preferably H or lower alkyl or lower alkoxy.

Preferred compounds of formula IV include those in which, when A represents CH, then G does not represent -C(O)OH.

Procedures for the conversion of selected groups which G may represent to certain -C(R^{x})=NH groups are known to those skilled in the art, and are described *inter alia* in: J. March, *Advanced Organic Chemistry,* 3^{rd} Edition, Chapter 10, 371-374, John Wiley & Sons **(1985);** and *Comprehensive Organic Functional Group Transformations,* edited by A. Katritzky, O. Meth-Cohn, and C. Rees, 1^{st} Edition, Volume 5, Sections 5.17 (page 653) and 5.19 (page 741), Pergamon Press **(1995),** the disclosures of which documents are hereby incorporated by reference. For example, compounds of formula II may be prepared by way of the following procedures.
1) For compounds of formula II in which R^{x} represents -OR^{e} (wherein R^{e} represents lower alkyl (optionally interrupted by O), alkylHet or alkylaryl, e.g. lower alkyl):
   (a) a corresponding compound of formula IV in which G represents -CN may be reacted with an alcohol of formula VA,

      R_{α}OH VA

      wherein R_{α} represents lower alkyl (optionally interrupted by O), alkylHet or alkylaryl (e.g. lower alkyl), and Het is as hereinbefore defined, in the presence of a suitable protic acid (e.g. HCl gas) and optionally in the presence of an appropriate solvent (e.g. diethyl ether, dioxan, benzene or chloroform). The skilled person will appreciate that such a reaction may be performed at low temperature (e.g. below 5°C);
   (b) a corresponding compound of formula IV in which G represents -C(O)NH₂ may be reacted with an appropriate alkylating agent of formula VB,

      R_{α}-Z¹ VB

      wherein Z¹ represents a leaving group such as halo, -OS(O)₂OR_{α}, -OS(O)₂CF₃ or OR_{α2}, and R_{α} is as hereinbefore defined, optionally in the presence of a suitable solvent (e.g. dichloromethane), followed by deprotonation of the resulting alkoxymethyleneiminium salt in the presence of a suitable base (e.g. NaOH or a tertiary amine such as triethylamine); or
   (c) a corresponding compound of formula IV in which G represents -C(OR_{α})₃, wherein R_{α} is as hereinbefore defined, may be reacted with ammonia, or an *N*-protected derivative thereof, for example in the presence of a catalytic quantity of a suitable acid (e.g. a protic acid such as *p*-toluenesulfonic acid), and optionally in the presence of an appropriate solvent (e.g. dichloromethane).
2) For compounds of formula II in which R^{x} represents -OR^{e} (wherein R^{e} represents Het or aryl, e.g. phenyl), a corresponding compound of formula IV in which G represents -CN may be reacted with a compound of formula VC,

   R_{β}OH VC

   wherein R_{β} represents Het or aryl (e.g. phenyl), and Het is as hereinbefore defined, for example in the presence of a suitable catalyst (e.g. a Lewis acid such as ZnCl₂ and/or a protic acid such as HCl) and optionally in the presence of an appropriate solvent (e.g. dichloromethane).
3) For compounds of formula II in which R^{x} represents -NH₂:
   (a) a corresponding compound of formula IV in which G represents -CN may be reacted with hydrazine, hydroxylamine or *O*-lower alkyl hydroxylamine, followed by reduction of the resultant intermediate under standard conditions (e.g. palladium-catalysed hydrogenation); or
   (b) a corresponding compound of formula IV in which G represents -C(=NOR^{f})N(R^{e})₂, wherein R^{f} is as hereinbefore defined, may be reduced under standard conditions (e.g. palladium-catalysed hydrogenation).
4) For compounds of formula II in which R^{x} represents -NH₂, -NHR^{a} or -N(R^{b})R^{c}, a corresponding compound of formula IV in which G represents -CN may be reacted with a compound of formula VD,

   HN(R_{χ})(R_{δ}) VD

   wherein R_{χ} and R_{δ} independently represent H or R^{a}, and R^{a} is as hereinbefore defined, for example in the presence of a suitable catalyst (e.g. a copper(I) salt such as CuCl) and optionally in the presence of an appropriate solvent (e.g. dimethylsulfoxide or a lower alkyl alcohol such as methanol or ethanol).
5) For compounds of formula II in which R^{x} represents -SH:
   (a) a corresponding compound of formula IV in which G represents -CN may be reacted with hydrogen sulfide, for example in the presence of a suitable base (e.g. a tertiary amine such as triethylamine) and optionally in the presence of an appropriate solvent (e.g. a lower alkyl alcohol such as ethanol); or
   (b) a corresponding compound of formula IV in which G represents -C(O)NH₂ may be reacted with a reagent that effects oxygen-sulfur exchange (e.g. P₄S₁₀ or Lawesson's reagent), for example in the presence of an appropriate solvent (e.g. toluene).
6) For compounds of formula II in which R^{x} represents -SR^{d}, a corresponding compound of formula IV in which G represents -CN may be reacted with a compound of formula VE,

   R^{d}SH VE

   wherein R^{d} is as hereinbefore defined, for example in the presence of a suitable base (e.g. a tertiary amine such as triethylamine) and optionally in the presence of an appropriate solvent (e.g. a lower alkyl alcohol such as ethanol).
7) For compounds of formula II in which R^{x} represents halo (e.g. chloro), a corresponding compound of formula IV in which G represents -C(O)NH₂ may be reacted with a suitable halogenating agent (e.g. a chlorinating agent such as PCl₅ or S(O)Cl₂), optionally in the presence of an appropriate solvent (e.g. benzene, CCl₄, CHCl₃ or dichloromethane).

Compounds of formula II may similarly be prepared from other compounds of formula II by reaction with a reagent that will convert one R^{x} group to another. In this respect, compounds of formula II may additionally be prepared by way of the following procedures.
I) For compounds of formula II in which R^{x} represents OR^{e} (wherein R^{e} represents lower alkyl, alkylHet or alkylaryl, e.g. lower alkyl), a corresponding compound of formula IT in which R^{x} represents Cl may be reacted with a compound of formula VA, as hereinbefore defined, for example in the presence of an appropriate solvent (e.g. dichloromethane) and a suitable base (e.g. an alkali metal alkoxide such as sodium ethoxide, or a tertiary amine such as triethylamine).
II) For compounds of formula II in which R^{x} represents -NH₂, -NHR^{a} or -N(R^{b})R^{c}, a corresponding compound of formula II in which R^{x} represents Cl, -SH, -SR^{d} or -OR^{e}, wherein R^{d} and R^{e} are as hereinbefore defined, may be reacted with an appropriate compound of formula VD, as hereinbefore defined, or an acid (e.g. hydrogen chloride or CH₃C(O)OH) addition salt thereof, for example optionally in the presence of an appropriate solvent (e.g. dichloromethane, ethanol, diethyl ether, dioxan, benzene or chloroform) and/or a suitable reaction promoter (for example: for reaction of compounds of formula II in which R^{x} represents -SH, a mercury(II) salt to act as a sulfide scavenger; and for reaction of compounds of formula II in which R^{x} represents -SR^{d}, a pH buffer (e.g. sodium acetate / acetic acid)).
III) For compounds of formula II in which R^{x} represents -SR^{d}, a corresponding compound of formula II in which R^{x} represents -SH may be reacted with a compound of formula VF,

   R^{d}-Z² VF

   wherein Z² represents a leaving group such as halo (e.g. iodo), alkanesulfonate, perfluoroalkanesulfonate (e.g. trifluoromethanesulfonate) or arenesulfonate (e.g. *p*-toluenesulfonate), and R^{d} is as hereinbefore defined, optionally in the presence of an appropriate solvent (e.g. dichloromethane or acetone) and/or a suitable base (e.g. a tertiary amine such as triethylamine).

Compounds of formula IV may be prepared via a variety of techniques. For example:
(a) Compounds of formula IV may be prepared by reaction of a compound of formula VI, 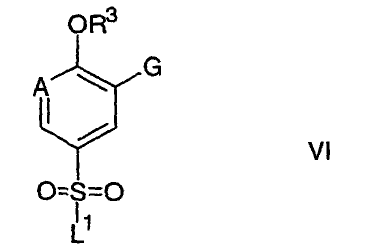 wherein L¹ is a leaving group (e.g. halo) and A, G and R³ are as hereinbefore defined, with a compound of formula VII, 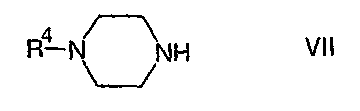 wherein R⁴ is as hereinbefore defined. This reaction may be performed at, for example, low temperatures (e.g. between -10°C and room temperature), in the presence of an appropriate solvent (e.g. a C₁₋₃ alcohol, ethyl acetate, dichloromethane, toluene or heptane), at least one equivalent of the compound of formula VII and, optionally, another suitable base (such as a base that does not react with or, if it does react, a base that further activates the sulfonyl chloride (for example: a tertiary amine such as triethylamine, *N*-ethyldiisopropylamine, 1,5-diazabicyclo[4.3.0]non-5-ene or 1,8-diazabicyclo[5.4.0]undec-7-ene; or a metal hydride, oxide, carbonate or bicarbonate)).
   Compounds of formula VI are available using known techniques. For example, compounds of formula VI may be prepared from a corresponding compound of formula VIII, 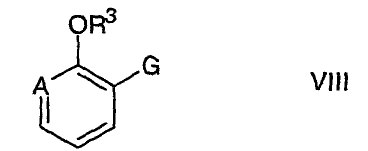 wherein A, G and R³ are as hereinbefore defined, for example using conventional methods for the introduction of a -SO₂L¹ group into an aromatic or heteroaromatic ring system, such as reaction of a compound of formula VIII, optionally in the presence of an appropriate solvent (e.g. dichloromethane), with a compound of formula L¹SO₃H and (optionally) a compound of formula SO(L¹)₂. When L¹ is chloro, reaction may take place at between 0°C and room temperature in the presence of an excess of chlorosulfonic acid (optionally in conjunction with an excess of thionyl chloride), and optionally in an appropriate organic solvent (e.g. dichloromethane).
(b) Compounds of formula IV in which G represents -CN or -C(O)NH₂ may be prepared by reaction of a compound of formula IX, 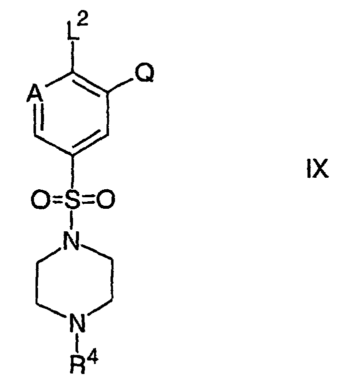 wherein Q represents -CN or -C(O)NH₂, L² represents a suitable leaving group and A and R⁴ are as hereinbefore defined, for example by reaction with a compound that will provide the group R³O (e.g. an alkoxide base). This route is preferred for the preparation of compounds of formula IV in which A represents N.
   Suitable leaving groups L² include standard groups known to those skilled in the art, such as optionally substituted arylsulfonyloxy groups (e.g. *p*-toluenesulfonyloxy), optionally substituted C₁₋₄ alkanesulfonyloxy groups (e.g. methanesulfonyloxy, trifluoromethanesulfonyloxy), halosulfonyloxy (e.g. fluorosulfonyloxy), halonium, diarylsulfonylamino (e.g. ditosyl), quaternary ammonium C₁₋₄ alkylsulfonyloxy, C₁₋₄ perfluoroalkanoyloxy (e.g. trifluoroacetyloxy), C₁₋₄ alkanoyloxy (e.g. acetyloxy), aroyloxy (e.g. benzoyloxy), diazonium, oxonium or perchloryloxy groups.
   More preferred values of L² include a different lower alkoxy group to that which is to be replaced by the group R³O (e.g. methoxy, provided that R³ does not represent methyl) and, especially, halo (including bromo and, particularly, chloro).
   The skilled person will appreciate that compounds that may serve to provide the group R³O include lower alkoxides of alkali metals (e.g. lithium, sodium, potassium), or of alkaline earth metals (e.g. magnesium, calcium). Preferred alkoxides include those of sodium and potassium.
   Alternatively, the R³O⁻ anion may be produced *in situ* by reaction of the relevant lower alkyl alcohol (or an alkali/alkaline earth metal alkoxide) with an auxiliary base, which should not compete with the relevant R³O⁻ group in the nucleophilic substitution of L² by being suitably sterically hindered. In this respect, suitable auxiliary bases may include a sterically hindered alkoxide or a secondary or tertiary amine.
   Compounds of formula IX may be prepared by reaction of a compound of formula X, 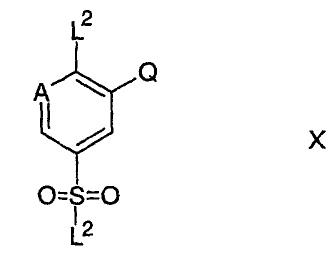 wherein A, Q and L² are as hereinbefore defined with a compound of formula VII as hereinbefore defined, for example as described hereinbefore.
   Compounds of formula X may be prepared by known techniques. For example compounds of formula X in which both L² groups represent halo (e.g. chloro) may be prepared by reaction of a corresponding compound of formula XI,
   wherein A and Q are as hereinbefore defined, with a suitable halogenating agent (e.g. thionyl chloride), for example at around 80 to 100°C, optionally in the presence of a suitable solvent (e.g. dimethylformamide) and/or (optionally) an appropriate activating agent (e.g. dimethylformamide). The skilled person will appreciate that when an activating agent and a solvent are both employed, they may be either the same or different compounds.
   Compounds of formula XI may be prepared by techniques known to those skilled in the art. For example, compounds of formula XI may be prepared by reacting a corresponding compound of formula XII,
   wherein A and Q are as hereinbefore defined, with a sulfonating agent (e.g. oleum) under conditions known to those skilled in the art.
(c) Compounds of formula IV in which G represents -CN may alternatively be prepared by dehydration of a corresponding compound of formula IV in which G represents -C(O)NH₂, under appropriate reaction conditions, for example at low temperature (e.g. at between -5°C and room temperature (preferably at around 0°C)) in the presence of a suitable dehydrating agent (for example: P₂O₅; POCl₃; PCl₅; CCl₄ and triphenylphosphine; trifluoroacetic anhydride and a suitable base (e.g. triethylamine or pyridine); or SOCl₂) and an appropriate organic solvent (e.g. dichloromethane, toluene, chlorobenzene or heptane).
(d) Compounds of formula IV in which G represents -C(O)NH₂ may be prepared from a corresponding compound of formula IV in which G represents -C(O)OH, for example by reaction with ammonia or a derivative thereof (e.g. ammonium acetate). The skilled person will appreciate that this reaction may preferably be carried out in the presence of an appropriate activating reagent (e.g. *N,N'*-carbonyldiimidazole), in an appropriate solvent, e.g. ethyl acetate, dichloromethane or butan-2-one, resulting in the formation of an intermediate imidazolide (which may be isolated if desired), followed by reaction with e.g. ammonium acetate at between room and reflux temperature. Those skilled in the art will also appreciate that the activation of benzoic acid derivatives may also be accomplished with many other activating agents, for example as described in J. March, *Advanced Organic Chemistry,* 3^{rd} Edition, Chapter 10, 371-374, John Wiley & Sons (**1985**).
(e) Compounds of formula IV in which G represents -C(O)OH may be prepared by known techniques. For example, such compounds may be prepared according to, or by analogy with, methods described in European patent application EP 812 845 (the disclosures in which document are hereby incorporated by reference). Compounds of formula IV in which G represents -C(O)OH may alternatively be prepared by reaction of a corresponding compound of formula XIII,
   wherein A and L² are as hereinbefore defined, with a compound that with provide the group R³O, for example under conditions as described hereinbefore for the preparation of compounds of formula IV.
   Compounds of formula XIII may be prepared by known techniques, for example, according to, or by analogy with procedures described hereinbefore for the preparation of compounds of formula IX.

Other compounds of formula IV may be prepared from appropriate starting materials (which include *inter alia* compounds of formula IV), using techniques known to those skilled in the art and/or according to, or by analogy with procedures described hereinbefore for the preparation of compounds of formula II.

Compounds of formula IIIA, IIIB, VA, VB, VC, VD, VE, VF, VII, VIII, XII, and derivatives thereof, when not commercially available or not subsequently described, may be obtained either by analogy with processes described herein, or by conventional synthetic procedures, in accordance with standard techniques, from readily available starting materials using appropriate reagents and reaction conditions.

Compounds may be isolated from reaction mixtures using known techniques.

Substituents on the aryl (e.g. phenyl), and (if appropriate) heterocyclic, group(s) in compounds defined herein may be converted to other substituents using techniques well known to those skilled in the art. For example, amino may be converted to amido, amido may be hydrolysed to amino, hydroxy may be converted to alkoxy, alkoxy may be hydrolysed to hydroxy etc.

It will be appreciated by those skilled in the art that, in the processes described above, the functional groups of intermediate compounds may be, or may need to be, protected by protecting groups.

Functional groups which it is desirable to protect thus include hydroxy, amino and carboxylic acid. Suitable protecting groups for hydroxy include trialkylsilyl and diarylalkylsilyl groups (e.g. *tert-*butyldimethylsilyl, *tert*-butyldiphenylsilyl or trimethylsilyl), tetrahydropyranyl, benzyl and alkylcarbonyl groups (e.g. methyl- and ethylcarbonyl groups). Suitable protecting groups for amino include benzyl, *tert-*butyloxycarbonyl, 9-fluorenylmethoxycarbonyl or benzyloxycarbonyl. Suitable protecting groups for carboxylic acid include C₁₋₆ alkyl, allyl or benzyl esters.

The protection and deprotection of functional groups may take place before or after any of the reaction steps described hereinbefore.

Protecting groups may be removed in accordance with techniques which are well known to those skilled in the art and as described hereinafter.

The use of protecting groups is fully described in "Protective Groups in Organic Chemistry", edited by JWF McOmie, Plenum Press (1973), and ''Protective Groups in Organic Synthesis", 3^{rd} edition, TW Greene & PGM Wutz, Wiley-Interscience (1999).

Persons skilled in the art will appreciate that, in order to obtain compounds of the formula II in an alternative, and, on some occasions, more convenient, manner, the individual process steps mentioned herein may be performed in a different order, and/or the individual reactions may be performed at a different stage in the overall route (i.e. substituents may be added to and/or chemical transformations performed upon, different intermediates to those associated hereinbefore with a particular reaction). This will depend *inter alia* on factors such as the nature of other functional groups present in a particular substrate, the availability of key intermediates and the protecting group strategy (if any) to be adopted. Clearly, the type of chemistry involved will influence the choice of reagent that is used in the said synthetic steps, the need, and type, of protecting groups that are employed, and the sequence for accomplishing the synthesis.

Certain intermediates that are employed in the processes described herein are novel. According to the invention there is further provided: (a) compounds of formulae II as defined hereinbefore; and (b) compounds of formula IV as defined hereinbefore. Preferred compounds of formula II include those in which, when A represents CH, then R^{x} does not represent -NH₂.

According to a further aspect of the invention there is provided compounds of formula II, as defined hereinbefore, in which R^{x} represents -SR^{d}, -SH and -OR^{e} (wherein R^{d} and R^{e} are as hereinbefore defined).

The process of the invention may have the advantage that sildenafil and analogues thereof may be prepared from commercially-available starting materials in fewer steps than in processes described in the prior art, without concomitant losses in terms of yield of key intermediates and of final products.

Further, the process of the invention may have the advantage that sildenafil and analogues thereof may be prepared in less time, more conveniently, and at a lower cost, than when prepared in processes described in the prior art.

The invention is illustrated, but in no way limited, by the following examples.

All ¹H NMR spectra were recorded using a Varian Unity 300 MHz machine.

### Example 1

### 5-[2-Ethoxy-5-(4-methylpiperazin-1-ylsulfonyl)phenyl]-1-methyl-3-n-propyl-1,6-dihydro-7H-pyrazolo[4,3-d]pyrimidin-7-one, sildenafil

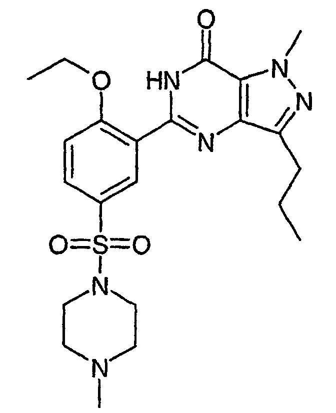

A solution of ethyl 2-ethoxy-5-(4-methyl-1-piperazinylsulfonyl)-benzimidate (2.2 g, 6.2 mmol, from step 1(c) below) and 4-amino-1-methyl-3-propyl-1*H*-pyrazole-5-carboxamide (Example 37 of EP 0463756) (17 mL of a 10% w/v solution in ethyl acetate, 6.8 mmol) in xylene (40 mL) was heated to reflux. Approximately 10 mL of the solvent was distilled to remove the ethyl acetate. The reaction started to foam at 129°C and gave off ammonia gas. The temperature gradually rose to 136°C over three days and the foaming ceased. When the reaction was complete, the solvent was removed to give a brown oil which was further purified using medium pressure column chromatography (DCM and methanol as eluents), giving the title product in 76% yield, along with recovered 4-amino-1-methyl-3-propyl-1*H*-pyrazole-5-carboxamine (22.6%). The yield based upon unrecovered 4-amino-1-methyl-3-propyl-1*H*-pyrazole-5-carboxamide was 85%. The product was recrystallised from methyl ethyl ketone (MEK) to give material for analysis.

mp 184-185°C
¹H NMR (CDCl₃) δ 1.03 (3H, t), 1.62 (3H, t), 1.85 (2H, m), 2.22 (3H, s), 2.49 (4H, m), 2.94 (2H, t), 3.11 (4H, m), 4.27 (3H, s), 4.38 (2H, q), 7.17 (1H, d), 7.83 (1H, d), 8.81 (1H, s)
m/z found 475 [M+H]⁺ 100%, C₂₂H₃₁N₆O₄S requires 475

### Preparative Examples for Example 1

### 1(a) 5-Chlorosulfonyl-2-ethoxybenzonitrile (Compound of formula VI where A=CH; R³=Et; G=CN; L=Cl)

Commercially available 2-ethoxybenzonitrile (1.0 g, 0.007 mol) was added to an ice-cold solution of chlorosulfonic acid (1.9 mL, 0.03 mol) and thionyl chloride (0.5 mL, 0.007 mol) over 30 minutes, left to stir overnight, quenched by pouring onto ice/water (20 mL), and granulated for 30 minutes. The precipitated product was filtered off, washed with water and dried on the filter under nitrogen to give 1.0 g (59%) of the sub-title compound as a yellow solid, which was used directly in the next step.

¹H NMR (CDCl₃) δ 1.54 (3H, t), 4.31 (2H, m), 7.16 (1H, d), 8.18 (1H, d), 8.12 (1H, s)

### 1(b) 2-Ethoxy-5-(4-methyl-1-piperazinylsulfonyl)benzonitrile (compound of formula IV where A=CH; R³=Et; G=CN; R⁴=Me)

5-Chlorosulfonyl-2-ethoxybenzonitrile (1.0 g, 0.004 mol, from step 1(a) above) was dissolved in dichloromethane (DCM; 6 mL) and cooled to between 0 and 5°C. *N-*Methylpiperazine (0.8 mL, 0.0091 mol) was added dropwise over 30 minutes and the solution left to stir overnight. The solution was diluted with 10 mL of water and the product extracted with dichloromethane. The solvent was removed *in vacuo* to give the sub-title compound as a yellow oil (0.8 g, 66.6%).

¹H NMR (CDCl₃) δ 1.54 (3H, t), 2.2 (3H, s), 2.45 (4H, m), 2.97 (4H, m), 4.18 (2H, q), 7.07 (1H, d), 7.84 (1H, d), 7.90(1H, s)
m/z found 310 [M+H⁺]75%, C₁₄H₁₉N₃O₃S requires 310

### 1(c) Ethyl 2-ethoxy-5-(4-Methyl-1-piperazinylsulfonyl)benzimidate (compound of formula II where A=CH; R³=Et: R⁴=Me)

A suspension of 2-ethoxy-5-(4-methyl-1-piperazinylsulfonyl)benzonitrile (2.6 g, 8.4 mmol, from step 1(b) above) in ethanol (80 mL) was cooled to 0° C. HCl gas was slowly bubbled through the resultant until saturation. After standing for 3 days, the reaction was complete and ethanol was removed *in vacuo.* The crude solid was dissolved in DCM and washed with aqueous sodium bicarbonate solution. The solvent was removed to give the sub-title compound as a brown solid (48%).

mp 158-160°C
¹H NMR (CDCl₃) δ 1.39 (3H, t), 1.56 (3H, t), 2.53 (3H, s), 2.89 (4H, m), 3.32 (4H, m), 4.14 (2H, q), 4.37 (2H, q), 7.08 (1H, d), 7.74 (1H, d), 8.15 (1H, s)
m/z found 357 [M+H]⁺ 25%, C₁₆H₂₆N₃O₄S requires 357

### 1(d) Alternative Synthesis of 2-Ethoxy-5-(4-methyl-1-piperazinylsulfonyl)-benzonitrile - the compound of Step 1(b) above

### 1(d)(i) 2-Ethoxy-5-(4-methyl-1-piperazinylfonyl)benzamide (compound of formula II where A=CH; R³=Et; G=C(O)NH₂;R⁴=Me)

To a slurry of 2-ethoxy-5-(4-methyl-1-piperazinylsulfonyl)benzoic acid (50 g, 0.15 mol, see EP 812 845) in EtOAc (250 mL), was added *N,N'*-carbonyldiimidazole (CDI; 27 g, 0.166 mol) in one portion. The slurry was heated to about 40°C, upon which the reaction commenced. After the CDI had reacted, the reaction was heated to reflux for 4 hours. Ammonium acetate (40 g, 0.5 mol) was added to the slurry, and the resultant was left to reflux overnight. After cooling the resultant slurry was filtered to give the sub-title product as a fine white solid (40.7 g, 83%).

mp 185-189°C
¹H NMR (CDCl₃) δ 1.58 (3H, t), 2.29 (3H, s), 2.47 (4H, m), 3.06 (4H, m), 4.29 (2H, q), 6.13 (1H, s), 7.12 (1H, d), 7.70 (1H, s), 7.82 (1H, d), 8.60 (1H, s)
m/z found 328 [M+H⁺]100%, C₁₄H₂₂N₃O₄S requires 328

### 1(d)(ii) 2-Ethoxy-5-(4-methyl-1-piperazinylsulfony)benzonitrile (compound of formula II where A=CH; R³=Et;G= CN; R⁴=Me)

To an ice cold suspension of 2-ethoxy-5-(4-methyl-1-piperazinylsulfonyl)benzamide (32 g, 0.098 mol, from step 1(d)(i) above) and triethylamine (56 mL, 0.38 mol) in DCM was added trifluoroacetic anhydride (34.4 mL, 0.22 mol) which resulted in a brown solution. This was allowed to stir overnight and was quenched with water (100 mL). The organic layer was washed with water (2 x 100 mL) and brine (50 mL) and the DCM was removed *in vacuo* to give 56.8 g of brown oil which was recrystallised from ethyl acetate to give the product as a brown solid (18.7 g, 61.7%).

mp 119-120°C
¹H NMR (CDCl₃) δ 1.54 (3H, t), 2.2 (3H, s), 2.45 (4H, m), 2.97 (4H, m), 4.18 (2H, q), 7.07 (1H, d), 7.84 (1H, d), 7.90(1H, s)
m/z found 310 [M+H⁺] 75%, C₁₄H₁₉N₃O₃S requires 310

The product of Preparative example 1(d) may be used to prepare compounds of formula II, and of formula I, in which R³ is ethyl and R⁴ is methyl, by employing similar procedures to those described in Example 1, and in the preparative Example step 1(c).

### Example 2

### Alternative Synthesis of (5-[2-ethoxy-5-(4-methylpiperazin-1-ylsulfonyl)phenyl]-1-methyl-3-n-propyl-1,6-dihydro-7H-pyrazolo[4,3-d]pyrimidin-7-one), Sildenafil.

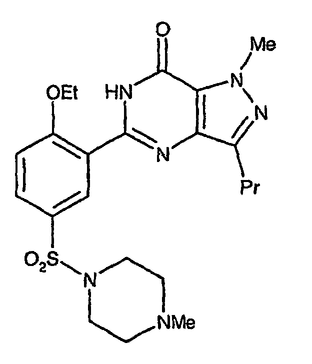

4-Amino-1-methyl-3-*n*-propyl-1*H*-pyrazole-5-carboxylate (prepared according to Example 37 of EP-0463756) (0.182 g, 1.0 mMol) and 2-ethoxy-5-(4-methyl-1-piperazinylsulfonyl)benzamidine (the compound of Preparation 5 hereinafter) (0.326 g, 1.0 mMol) were stirred in xylene (15 mL) and this mixture was heated to reflux for 9h. After evaporation and re-evaporation from toluene the residue was subjected to chromatography on silica gel eluting with ethyl acetate / methanol mixtures to give the desired product, 0.033g. M/Z = 475 (M+H); 1H NMR (300MHz, CDCl3); 1.04 (t, 3H), 1.66 (t, 3H), 1.88 (sextet, 2H), 2.29 (s, 3H), 2.51 (m, 4H), 2.95 (t, 3H), 3.13 (m, 4H), 4.29 (s, 3H), 4.39 (quart. 2H), 7.16 (d, 1H), 7.85 (dd, 1H), 8.85 (d, 1H).

According to a preferred process according to the present invention there is provided a process for the preparation of sildenafil substantially as described in Examples 1 and 2 herein before and Preparations for Example 1 herein before.

According to another preferred process compounds wherein G=CO₂Et can be prepared via the telescoped chemistry of Preparation 2 as detailed hereinafter.

Example 3 illustrates how one of the preferred compounds noted herein can be made. The present invention provides an alternative method for the preparation of the compound of Example 3 by using any of the routes hereinbefore detailed and especially as described in Examples 1 and 2.

### Example 3

### 2-(Methoxyethyl)-5-[2-ethoxy-5-(4-ethylpiperazin-1-ylsulphonyl)pyridin-3-yl]-3-ethyl-2,6-dihydro-7H-pyrazolo[4,3-d]pyrimidin-7-one

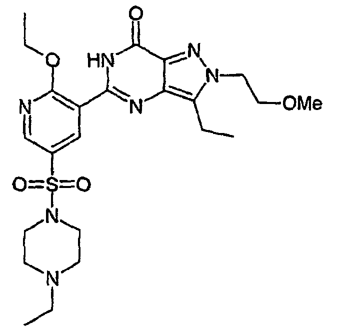

A mixture of the product from step 3(j) below (0.75mmol), potassium bis(trimethylsilyl)amide (298mg, 1.50mmol) and ethyl acetate (73 microlitres, 0.75mmol) in ethanol (10ml) was heated at 120°C in a sealed vessel for 12 hours. The cooled mixture was partitioned between ethyl acetate and aqueous sodium bicarbonate solution, and the layers separated. The organic phase was dried (MgSO₄), and evaporated under reduced pressure. The crude product was purified by column chromatography on silica gel using dichloromethane:methanol (98:2) as eluant to afford the title compound, 164mg; Found : C, 53.18; H, 6.48; N, 18.14; C₂₃H₃₃N₇O₅S;0.20C₂H₅CO₂CH₃ requires C, 53.21; H, 6.49; N, 18.25%; δ (CDCl₃): 1.04 (3H, t), 1.40 (3H, t), 1.58 (3H, t), 2.41 (2H, q), 2.57 (4H, m), 3.08 (2H, q), 3.14 (4H, m), 3.30 (3H, s), 3.92 (2H, t), 4.46 (2H, t), 4.75 (2H, q), 8.62 (1H, d), 9.04 (1H, d), 10.61 (1H, s); LRMS : m/z 520 (M+1)⁺ ; mp 161-162°C.

### Preparation of Starting Materials for Example 3

### 3(a) Pyridine-2-amino-5-sulphonic acid

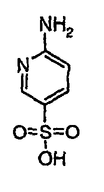

2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the sub-title compound as a solid, 111.3g; LRMS : m/z 175 (M+1)⁺.

### 3(b) Pyridine-2-amino-3-bromo-5-sulphonic acid

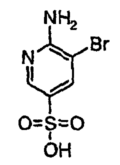

Bromine (99g, 0.62mol) was added dropwise over an hour, to a hot solution of the product from step 3(a) (108g, 0.62mol) in water (600ml) so as to maintain a steady reflux. Once the addition was complete the reaction was cooled and the resulting mixture filtered. The solid was washed with water and dried under suction to afford the sub-title compound, 53.4g; δ (DMSOd₆, 300MHz): 8.08 (1H, s), 8.14 (1H, s); LRMS : m/z 253 (M)⁺.

### 3(c) Pyridine-3-bromo-2-chloro-5-sulphonyl chloride

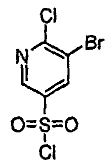

A solution of sodium nitrite (7.6g, 110.0mmol) in water (30ml) was added dropwise to an ice-cooled solution of the product from step 3(b) (25.3g, 100.0mmol) in aqueous hydrochloric acid (115ml, 20%), so as to maintain the temperature below 6°C. The reaction was stirred for 30 minutes at 0°C and for a further hour at room temperature. The reaction mixture was evaporated under reduced pressure and the residue dried under vacuum at 70°C for 72 hours. A mixture of this solid, phosphorus pentachloride (30.0g, 144mmol) and phosphorus oxychloride (1ml, 10.8mmol) was heated at 125°C for 3 hours, and then cooled. The reaction mixture was poured onto ice (100g) and the resulting solid filtered, and washed with water. The product was dissolved in dichloromethane, dried (MgSO₄), and evaporated under reduced pressure to afford the sub-title compound as a yellow solid, 26.58g; δ (CDCl₃, 300MHz) : 8.46 (1H, s), 8.92 (1H, s).

### 3(d) 3-Bromo-2-chloro-5-(4-ethylpiperazin-1-ylsulphonyl)pyridine

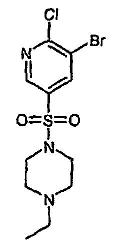

A solution of 1-ethylpiperazine (11.3ml, 89.0mmol) and triethylamine (12.5ml, 89.0mmol) in dichloromethane (150ml) was added dropwise to an ice-cooled solution of the product from step 3(c) (23.0g, 79.0mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the sub-title compound as an orange solid, 14.5g; δ (CDCl₃, 300MHz) : 1.05 (3H, t), 2.42 (2H, q), 2.55 (4H, m), 3.12 (4H, m), 8.24 (1H, s), 8.67 (1H, s).

### 3(e) 3-Bromo-2-ethoxy-5- 4-ethylpiperazin-1-ylsulphonyl)pyridine

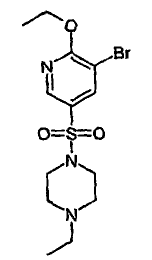

A mixture of the product from stage 3(d) (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the sub-title compound as a brown solid, 6.41g; Found : C, 41.27; H, 5.33; N, 11.11. C₁₃H₂₀BrN₃O₃S requires C, 41.35; H, 5.28; N, 10.99%; δ (CDCl₃, 300MHz) : 1.06 (3H, t), 1.48 (3H, t), 2.42 (2H, q), 2.56 (4H, m), 3.09 (4H, m), 4.54 (2H, q), 8.10 (1H, s), 8.46 (1H, s); LRMS : m/z 378, 380 (M+1)⁺.

### 3(f) Pyridine 2-ethoxy-5-(4-ethylpiperazin-1-ylsulphonyl)-3-carboxylic acid ethyl ester

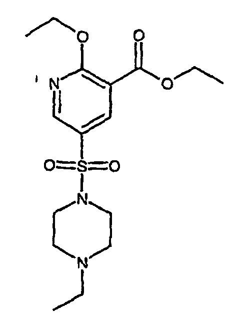

A mixture of the product from stage 3(e) (6.40g, 16.92mmol), triethylamine (12ml, 86.1mmol), and palladium (0) tris(triphenylphosphine) in ethanol (60ml) was heated at 100°C and 200 psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the sub-title compound as an orange oil, 6.2g; δ (CDCl₃, 300MHz) : 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s); LRMS : m/z 372 (M+1)⁺

### 3(g) Pyridine 2-ethoxy-5-(4-ethylpiperazin-1-ylsulphonyl)-3-carboxylic acid

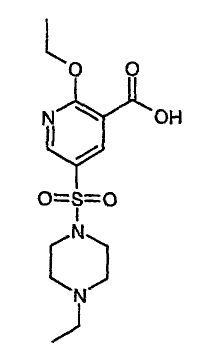

A mixture of the product from stage 3(f) (4.96g, 13.35mmol) and aqueous sodium hydroxide solution (25ml, 2N, 50.0mmol) in ethanol (25ml) was stirred at room temperature for 2 hours. The reaction mixture was concentrated under reduced pressure to half it's volume, washed with ether and acidified to pH 5 using 4N hydrochloric acid. The aqueous solution was extracted with dichloromethane (3x30ml), the combined organic extracts dried (MgSO₄) and evaporated under reduced pressure to afford the sub-title compound as a tan coloured solid, 4.02g; δ (DMSOd₆, 300MHz) : 1.18 (3H, t), 1.37 (3H, t), 3.08 (2H, q), 3.17-3.35 (8H, m), 4.52 (2H, q), 8.30 (1H, s), 8.70 (1H, s).

### 3(h) 4-[2-Ethoxy-5-(4-ethylpiperazin-1-ylsulphonyl)pyridin-3-ylcarboxamido]-1H-3-ethylpyrazole-5-carboxamide

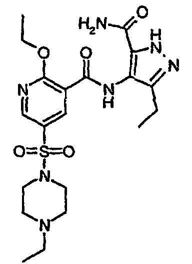

A solution of 4-amino-3-ethyl-1H-pyrazole-5-carboxamide (WO 9849166, preparation 8) (9.2g, 59.8mmol) in N,N-dimethylformamide (60ml) was added to a solution of the product from stage g) (21.7g, 62.9mmol), 1-hydroxybenzotriazole hydrate (10.1g, 66.0mmol) and triethylamine (13.15ml, 94.3mmol) in dichloromethane (240ml). 1-(3-Dimethylaminopropyl)-3-ethylcarbodiimide hydrochloride (13.26g, 69.2mmol) was added and the reaction stirred at room temperature for 6 hours. The dichloromethane was removed under reduced pressure, the remaining solution poured into ethyl acetate (400ml), and this mixture washed with aqueous sodium bicarbonate solution (400ml). The resulting crystalline precipitate was filtered, washed with ethyl acetate and dried under vacuum, to afford the sub-title compound, as a white powder, 22g; δ (CDCl₃+1 drop DMSOd₆) 0.96 (3H, t), 1.18 (3H, t), 1.50 (3H, t), 2.25-2.56 (6H, m), 2.84 (2H, q), 3.00 (4H, m), 4.70 (2H, q), 5.60 (1H, br s), 6.78 (1H, br s), 8.56 (1H, d), 8.76 (1H, d), 10.59 (1H, s), 12.10-12.30 (1H, s); LRMS: m/z 480 (M+1)⁺.

### 3(i) 2-Methoxyethyl-4-[2-ethoxy-5-(4-ethylpiperazin-1-ylsulphonyl)pyridin-3-ylcarboxamidol-3-ethylpyrazole-5-carboxamide

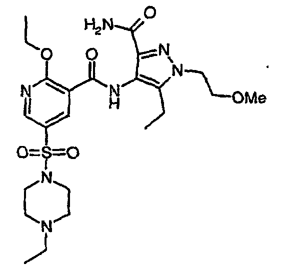

1-Bromo-2-methoxyethane (1.72mmol) was added to a solution of the product from stage 3(h) (750mg, 1.56mmol) and caesium carbonate (1.12g, 3.44mmol) in N,N-dimethylformamide (15ml) and the reaction stirred at 60° C for 18 hours. The cooled mixture was partitioned between water and ethyl acetate, and the layers separated. The organic layer was dried (MgSO₄), concentrated under reduced pressure and azeotroped with toluene to give a solid. This product was recrystallised from ether, to afford the sub-title compound as a white solid.

δ(CDCl₃): 1.04 (3H, t), 1.22 (3H, t), 1.60 (3H, t), 2.44 (2H, q), 2.54 (4H, m), 2.96 (2H, q), 3.12 (4H, m), 3.36 (3H, s), 3.81 (2H, t), 4.27 (2H, t), 4.80(2H, q), 5.35(1H, s), 6.68 (1H, s), 8.66 (1H, d), 8.86 (1H, d), 10.51 (1H, s).

### General Preparative Examples

### Preparation 1

### 2-Ethyl-2-ethoxy-5-(4-ethyl-1-piperazinylsulfonyl)pyridinoate - Compound IV wherein R³ & R⁴ = Et; A = N; G = CO₂₋H

### Preparation (1a) 2-Hydroxy-5-sulfonicotinic acid

2-Hydroxynicotinic acid (27Kg, 194.2mol) was added portionwise to 30% oleum (58.1Kg) at 50°C over 1hr. This caused an exotherm to 82°C. The reaction mixture was heated further to 140°C. After maintaining this temperature for 12hrs the reactor contents were cooled to 15C and filtered. The filter cake was then re-slurried with acetone (33Kg) at room temperature, filtered and dried to afford the sub-title compound (35.3Kg, 83%) as a white solid. Decomposition pt 273°C. δ (DMSO_{d6}): 7.93 (1H, d), 8.42 (1H, d). m/z (Found:220 [M+H]⁺, 100%. C₆H₆NO₆S requires 220.17).

### Preparation (1b) Ethyl 2-hydroxy-5-sulfonicotinoate

2-Hydroxy-5-sulfonicotinic acid (500g, 2.28mol) was dissolved in ethanol (2.5L) with stirring and heated to 80°C. After 30mins 0.5L of solvent was distilled off, then replaced with fresh ethanol (0.5L) and taken back to 80°C. After a further 60mins 1.0L of solvent was distilled off, then replaced with fresh ethanol (1.0L) and taken back to 80°C. After a further 60mins 1.0L of solvent was distilled off, the reaction cooled to 22°C and stirred for 16hr.

The precipitated product was filtered, washed with ethanol (0.5L) and dried at 50°C under vacuum to afford the sub-title compound (416g, 74%) as a white solid. Decomposition pt 237°C. δ (DMSO_{d6}): 1.25 (3H, t), 4.19 (2H,q), 7.66 (1H, d), 8.13 (1H, d). m/z (Found:248 [M+H]⁺, 100%. C₈H₁₀NO₆S requires 248.22).

### Preparation (1c) Ethyl 2-chloro-5-chlorosulfonicotinoate

Ethyl 2-hydroxy-5-sulfonicotinoate (24.7g, 0.1mol) was slurried in thionyl chloride (238g, 2.0mol) and dimethylformamide (1.0mL) with stirring. The reaction mixture was then heated to reflux for 2.5hr. The bulk of the thionyl chloride was removed under vacuum with residual thionyl chloride removed with a toluene azeotrope to afford the crude sub-title compound (30.7g, 108%) as a yellow oil. δ (CDCl₃): 1.46 (3H, t), 4.50 (2H, q), 8.72 (1H, d), 9.09 (1H, d). This was taken directly onto the next step.

### Preparation (1d) Ethyl 2-chloro-5-(4-ethyl-1-piperazinylsulfonyl)nicotinoate

Crude ethyl 2-chloro-5-chlorosulfonicotinoate (30.7g, 0.1mol assumed) was dissolved in ethyl acetate (150mL) with stirring then ice cooled. To this was added a solution of N-ethylpiperazine (11.4g, 0.1mol) and triethylamine (22.5g, 0.22mol) in ethyl acetate (50mL), carefully over 30mins, keeping the internal temperature below 10°C. Once the addition was complete the reaction was allowed to warm to 22°C and stir for 1hr. The solid was filtered off and the remaining filtrate was concentrated under vacuum to afford the crude sub-title compound (37.1g, 103%) as a crude yellow gum. δ (CDCl₃): 1.10 (3H, t), 1.42 (3H, m), 2.50 (2H, m), 2.60 (4H, m), 3.19 (4H, m), 4.43 (2H, q), 8.40 (1H, d), 8.80 (1H, d). m/z (Found:362 [M+H]⁺, 100%. C₁₄H₂₁ClN₃O₄S requires 362.85).

### Preparation (1e) Ethyl 2-ethoxy-5-(4-ethyl-1-perazinylsulfonyl)nicotinoate

A solution of ethyl 2-chloro-5-(4-ethyl-1-piperazinylsulfonyl)nicotinoate (36.1g, 0.1mol) in ethanol (180mL) was cooled to 10°C with stirring. Sodium ethoxide (10.2g, 0.15mol) was added portionwise keeping the temperature below 20°C. The reaction mixture was then stirred at ambient temperature for 18 hours. The precipitate was filtered off and water (180mL) added to the filtrate. The filtrate was then heated to 40°C for 1 hour. Ethanol (180mL) was then distilled off at ambient pressure and the remaining aqueous solution allowed to cool to ambient temperature. The precipitated product was then filtered off, washed with water and dried under vacuo at 50°C to afford the sub-title compound (12.6g, 34%) as a light brown solid. M.p. 66-68°C. δ (CDCl₃): 1.04 (3H, t), 1.39 (3H, t), 1.45 (3H, t), 2.41 (2H, q), 2.52 (4H, m), 3.08 (4H, m), 4.38 (2H, q), 2.57 (2H, q), 8.38 (1H, d), 8.61 (1H, d). m/z (Found: 372 [M+H]⁺, 100%. C₁₆H₂₆N₃O₅S requires 372.46).

### Preparation (1f) 2-Ethoxy-5-(4-ethyl-1-piperazinylsulfonyl)nicotinic acid

Ethyl 2-ethoxy-5-(4-ethyl-1-piperazinylsulfonyl)nicotinioate (10.2g, 0.0275mol) was dissolved in toluene (50mL) and a solution of sodium hydroxide (1.1g, 0.0275mol) in water (20mL) added to it. This two phase mixture was then stirred vigorously at ambient temperature overnight. The aqueous phase was separated off and adjusted to pH=5.6 by addition of conc. hydrochloric acid. The precipitated product was slurried with ice cooling for 15minutes, filtered, water washed and dried under vacuo at 50°C to afford the sub-title compound (4.1g, 43%) as an off-white solid. Mpt 206-207°C. δ (CDCl₃): 1.25 (3H, t), 1.39 (3H, t), (2H, q), 3.03 (4H, m), 3.25 (4H, m), 4.50 (2H, q), 8.25 (1H, d), 8.56 (1H, d). m/z (Found:344 [M+H]⁺, 100%. C₁₄H₂₂N₃O₅S requires 344.38).

This step is a simple hydrolysis and the yield of 43% is not optimum. The same hydrolysis was carried out in preparation 23 of PCT/IB99100519 (which is incorporated herein by reference) and a more optimised yield of 88% was obtained for the hydrolysis.

### Preparation (1g) Alternative Preparation for 2-Ethoxy-5-(4-ethyl-1-piperazinylsulfonyl)nicotinic acid (the compound of Preparation (1f) - Telescoped process in toluene from ethyl 2-hydroxy-5-sulfonicotinoate

Ethyl 2-hydroxy-5-sulfonicotinoate (the compound of Preparation (1b)) (441.5g, 1.79mol) was dissolved in toluene (1.77L) and thionyl chloride (1.06Kg, 8.93mol) and dimethylformamide (71.3mL) were then added. The stirred suspension was then heated to reflux for 3 hours to yield a yellow solution. Thionyl chloride (2.87L) was then distilled with continual replacement with toluene (2.15L). The pale yellow solution was then cooled to 10°C and a stirred solution of N-ethylpiperazine (198.9g, 1.66mol) and triethylamine (392.2g, 3.88mol) in toluene (700mL) added dropwise over 90 minutes keeping the reaction mixture below 10°C . The reaction was stirred at ambient temperature for 18 hours then washed with water (2 x 700mL) and brine (2 x 350mL). The toluene phase was azeotropically dried by distilling off 1750mL which was continuously replaced by dry toluene (1750mL). The remaining brown solution was cooled to 10°C and sodium ethoxide (178.0g, 2.62mol) was added portionwise keeping the temperature below 10°C. The reaction was then stirred at 10°C for 1 hour then allowed to warm to ambient temperature and stirred for 18 hours. Sodium hydroxide (34.9g, *mol) dissolved in water (1.5L) was then added to the toluene mixture and the 2 phase mixture was vigorously stirred for 18 hours at 40°C. Once cooled to ambient temperature the aqueous phase was separated off. To this was added conc. hydrochloric acid to pH=3 which precipitated a light brown solid which was granulated for 2 hour with ice cooling. The precipitate was filtered washed with water (300mL) and dried under vacuo at 50°C to afford the sub-title compound (338.4g, 57.4%) as an off-white solid. Mpt 206-207°C. δ (CDCl₃): 1.25 (3H, t), 1.39 (3H, t), 2.82 (2H, q), 3.03 (4H, m), 3.25 (4H, m), 4.50 (2H, q), 8.25 (1H, d), 8.56 (1H, d). m/z (Found:344 [M+H]⁺, 100%. C₁₄H₂₂N₃O₅S requires 344.38).

### Preparation 2

### 2-ethoxy-5-(4-methyl-1-piperazinylsulfonyl)benzonitrile.

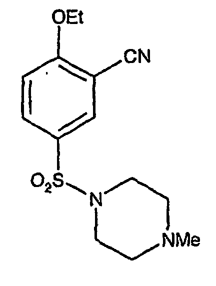

Triethylamine (49.1 g, 0.485 Mol) was added to a slurry of 2-ethoxy-5-(4-methyl-1-piperazinylsulfonyl)benzamide (40.9 g, 0.125 Mol) in dichloromethane (200 mL) and this mixture was cooled to 0°C. Trifluoroacetic anhydride (58.9 g, 0.28 Mol) was added dropwise over 45 min and was washed in with DCM (25 mL) before the reaction was stirred at ambient temperature for 18h. Water (125 mL) was added to the reaction with cooling. The layers were separated and the organic phase was washed with water before evaporation. The residue was stirred with ethyl acetate (150 mL) giving a crystalline solid which was filtered off and dried under vacuum; 27.4 g, 71%. m.p. = 130-131°C. M/Z = 310 (M+H); 1H NMR (300MHz, CDCl₃): 1.55 (t, 3H), 2.30 (s, 3H), 2,50 (m, 4H), 3.06 (m, 4H), 4.26 (quart. 2H), 7.08 (d, 1H), 7.90 (dd, 1H), 7.97 (d, 1H)

### Preparation 3

### 2-Ethoxy-N-hydroxy-5-[4-methylpiperazin-1-yl)sulfonyl]benzenecarboximidamidine

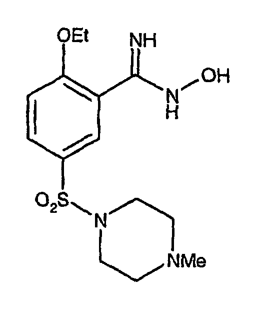

Hydroxylamine hydrochloride (20.8 g, 0.3 Mol) was added to a stirred slurry of 2-ethoxy-5-(4-methyl-1-piperazinylsulfonyl)benzonitrile (9.3 g, 0.03 Mol) in methanol (250 ml). To this mixture was added triethylamine (30.1 g, 0.3 Mol) this was washed into the reaction with methanol (50 mL) to give a solution. The reaction was allowed to stir for 90h at room temperature before evaporation to low volume. Water (500 mL) was added and after 30 min stirring, the title compound was filtered off, washed with water and dried under vacuum to yield 8.0 g, 78%. m.p. = 183-185°C (decomp.); M/Z = 343 (M+H). 1H NMR (300MHz, DMSO-d6): 1,38 (t, 3H), 2.14 (s, 3H), 2.50 (m, 4H), 3.30 (m, 4H), 4.20 (quart. 2H), 5.74 (s, 2H), 7.29 (dd, 1H), 7.70 (m, 2H), 9.61 (s, 1H).

### Preparation 4

### 1-{4-Ethoxy-3-[5-(triflouromethyl)-1,2,4-oxadiazol-3-yl]phenylsulfonyl}-4-methylpiperazine

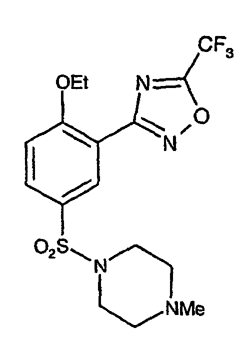

The *N*-hydroxyamidine prepared in preparation 3 (6.0 g, 0.0175 Mol) was added to trifluoroacetic acid (17.5 mL) at room temperature. Trifluoroacetic anhydride (17.5 mL) was then added to give a clear solution and after 2h stirring at room temperature the reaction was evaporated at reduced pressure. Toluene was added and then the mixture was re-evaporated. On stirring the residue with methanol the product crystallised and was filtered off, washed with methanol and dried under vacuum. This yielded 7.8 g 85% of the desired product; m.p. = 189-200°C (decomp.); M/Z = 421 (M+H). 1H NMR (300MHz, DMSO-d6): 1.42 (t, 3H), 2.77 (s, 3H), 3.29 (br, m, 4H), 3.43 (br, m, 4H), 4.37 (quart. 2H), 7.58 (d, 1H), 8.08 (dd, 1H), 8.23 (d, 1H).

### Preparation 5

### 2-ethoxy-5-(4-methyl-1-piperazinylsulfonyl)benzamidine

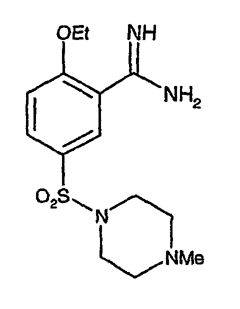

Water (4 ml) was added to a slurry of the compound prepared in preparation 4 (5.34 g, 0.01 Mol) in methanol (40 mL) at room temperature. The reaction mixture was then treated with triethylamine (2 mL) followed by Raney Ni (0.5 g). The resultant mixture was hydrogenated for 5h at ambient temperature. After the catalyst had been filtered off, the filtrate was heated to 90°C for 10 min and was then evaporated at reduced pressure. The residue was re-evaporated from toluene before being subjected to chromatography on silica gel eluting with toluene / methanol mixtures. Combination and evaporation of like fractions yielded 2.05 g (63%); M/Z = 327 (M+H); 1H NMR (500 MHz CDCl₃/CD₃OD): 1.41 (t, 3H), 2.60 (s, 3H), 2.99 (br, m, 4H), 3.20 (br, m, 4H), 4.16 (quart. 2H), 7.10 (d, 1H), 7.77 (d, 1H), 7.82 (dd, 1H).

### Preparation 6

### 2-ethoxy-5-(4-methyl-1-piperazinylsulfonyl)benzamidine (Preparation 5 above) can also be prepared by the following method;

Triethylaluminium (20 mL of 2M solution in hexane) was added to ammonium chloride (1.07 g, 0.2 Mol) slurried in toluene (20 mL) which had been pre-cooled to 5°C. The reaction was stirred without cooling until there was no further gas evolution when the compound prepared in preparation 2 (3.09 g, 0.1 Mol) was added. The mixture was stirred at 80°C for 40h. After cooling to room temperature silica gel (6 g) and dichloromethane (40 mL) were added and the mixture was stirred before filtration. The solids were washed with a methanol / dichloromethane mixture. The combined filtrate and washings were evaporated at reduced pressure to afford the product; 2.12 g 58%. Data as reported above.

### Preparation 7

### 2-ethoxy-5-(4-ethyl-1-piperazinylsulfonyl)benzonitrile.

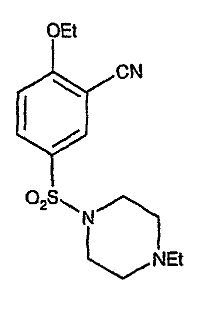

Commercially available 2-ethoxybenzonitrile (25 g, 0.17 Mol) was added dropwise to an ice cooled mixture of chlorosulfonic acid (50.8 mL, 0.765 Mol) and thionyl chloride (12.4 mL, 0.17 Mol) so as to keep the temperature below 10°C. The reaction was then stirred at ambient temperature for 18h before being poured onto ice/water. This mixture was stirred 1hr before the precipitated material was filtered off. The resultant solid was dissolved in acetone (300 mL) and triethylamine (25 mL, 0.179 Mol) was added followed by a slow addition of *N-*ethylpiperazine (25 mL, 0.198 Mol). After being left at ambient temperature for 65h the mixture was evaporated and the residue stirred with water (1 L) for 2h. The solids were filtered off, washed with water and dried before being chromatographed over silica gel using ethyl acetate methanol mixtures. Combination and evaporation of like fractions yielded 9.8 g, 17.8% of the title compound. Melting point = 86-88°C. M/Z = 324 (M+H). 300MHz Proton NMR (CDCl3); 1.06 (t, 3H), 1.55 (t, 3H), 2.43 (q, 2H), 2.54 (m, 4H), 3.06 (m, 4H), 4.25 (q, 2H), 7.08 (d, 1H), 7.90 (dd, 1H), 7.97 (d,1H).

### Preparation 8

### 2-ethoxy-5-(4-ethyl-1-piperazinylsulfonyl)benzamidine.

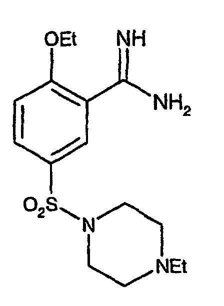

Trimethylaluminium (10 mL, 2 Molar solution in hexanes) was added dropwise to a slurry of ammonium chloride (1.07 g, 0.02 Mol) in toluene (15 mL) at 0°C. The mixture was stirred without cooling until gas evolution stopped. 2-ethoxy-5-(4-ethyl-1-piperazinylsulfonyl)benzonitrile (the compound of Preparation 7) (3.23 g, 0.01 Mol) was then added and was washed in with toluene (5 mL) before the reaction was stirred at 80°C for 40h. After cooling to room temperature silica gel (15 g) and dichloromethane (100 mL) were added. The mixture was then filtered and the solids were washed with dichloromethane / methanol mixtures. The combined filtrate and washings were evaporated and the residue was chromatographed over silica gel using dichloromethane methanol mixtures to afford 1.08 g, 28.6% of the title compound. M/Z = found 341 (M+H). 1H NMR (CD₃OD) 1.28 (t, 3H) 1.49 (t, 3H) 2.97 (q, 2H) 3.14 (br, s, 4H) 3.33(br, s, 4H) 4.32 (q, 2H) 7.45(d, 1H) 7.96 (d, 1H) 8.05 (dd, 1H).

The compounds of preparations 7 and 8 can also be used in the preparation of the compounds of published international application WO99/24433) according to a further aspect of the process of the present invention and in particular to prepare 2-[2-Ethoxy 5-(4-ethyl-piperazin-1-yl-1-sulphonyl)-phenyl]-5-methyl-7-propyl-3H-imidazo[5,1-*f*][1,2,4]triazin-4-one also known as 1-[[3-(3,4-dihydro-5-methyl-4-oxo-7-propylimidazo[5,1-*f*]]-*as*-triazin-2-yl)-4-ethoxyphenyl]sulphonyl] -4-ethylpiperazine (the compound of examples 20, 19, 337 and 336 of W099/24433).

## Claims

1. A process for the production of a compound of general formula I: 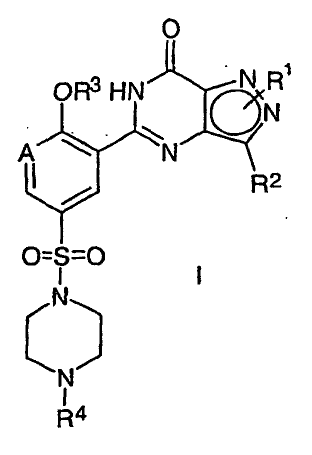 wherein
A represents CH or N;
R¹ represents H, lower alkyl (which alkyl group is optionally interrupted by O), Het, alkylHet, aryl or alkylaryl, which latter five groups are all optionally substituted (and/or, in the case of lower alkyl, optionally terminated) by one or more substituents selected from halo, cyano, nitro, lower alkyl, OR⁵, C(O)R⁶, C(O)OR⁷, C(O)NR⁸R⁹, NR^{10a}R^{10b} and SO₂NR^{11a}R^{11b};
R² and R⁴ independently represent lower alkyl;
R³ represents lower alkyl, which alkyl group is optionally interrupted by oxygen;
Het represents an optionally substituted four- to twelve-membered heterocyclic group, which group contains one or more heteroatoms selected from nitrogen, oxygen and sulfur; wherein Het groups containing N or S atoms may be present in their N- or S- oxidised form;
R⁵, R⁶, R⁷, R⁸, R⁹, R^{11a} and R^{11b} independently represent H or lower alkyl;
R^{10a} and R^{10b} either independently represent, H or lower alkyl or, together with the nitrogen atom to which they are attached, represent azetidinyl, pyrollidinyl or piperidinyl, wherein lower alkyl means C₁₋₆ alkyl which when there is a sufficient number of carbon atoms, may be linear or branched, be saturated or unsaturated, be cyclic, acyclic or part cyclic/acyclic, and/or be substituted by one or more halo atoms,
which process comprises the reaction of a compound of formula II, 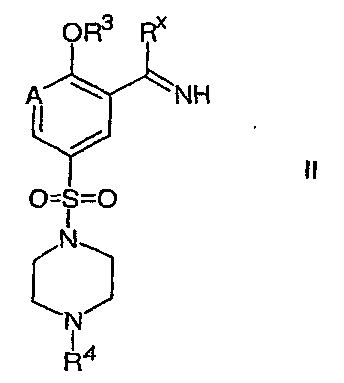 wherein R^{x} is a group substitutable by an aminopyrazole and A, R³ and R⁴ are as defined above,
with a compound of general formula III, 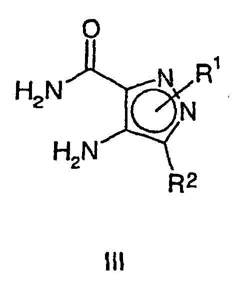 wherein R¹ and R² are as defined above.

2. A process as claimed in Claim 1, wherein, in the compound of general formula I, R¹ represents C₁₋₄ alkyl, which akyl group is optionally interrupted by an oxygen atom, and/or is optionally terminated by a Het group.

3. A process as claimed in Claim 2, wherein R¹ represents linear C₁₋₃ alkyl, which alkyl group is optionally interrupted by an oxygen atom, or is optionally terminated by a 2-pyridinyl group.

4. A process as claimed in any one of the preceding claims, wherein, in the compound of general formula I, R² represents C₁₋₄alkyl.

5. A process as claimed in Claim 4, wherein R² represents linear C₂₋₃ alkyl.

6. A process as claimed in any one of the preceding claims, wherein, in the compound of general formula I, R³ represents C₁₋₅ alkyl, which alkyl group is optionally interrupted by an oxygen atom.

7. A process as claimed in Claim 6, wherein R³ represents linear or branched C₂₋₄ alkyl, which alkyl group is optionally interrupted by an oxygen atom.

8. A process as claimed in any one of the preceding claims, wherein, in the compound of general formula I, R⁴ represents C₁₋₃ alkyl.

9. A process as claimed in Claim 8, wherein R⁴ represents C₁₋₂ alkyl.

10. A process as claimed in any one of the preceding claims, wherein the compound is selected from sildenafil, or any one of the following four compounds

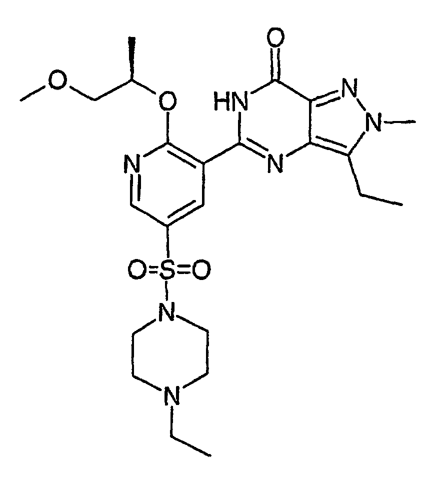

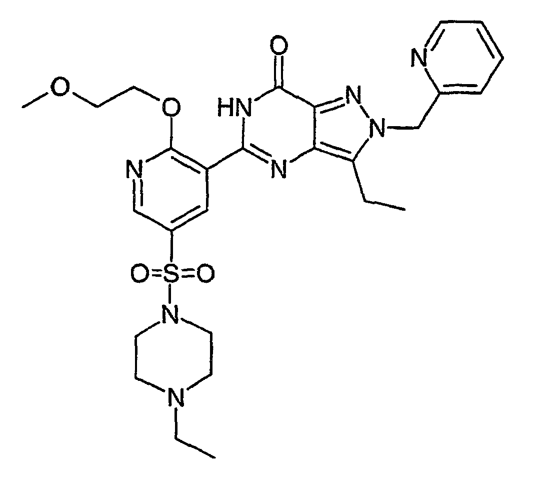

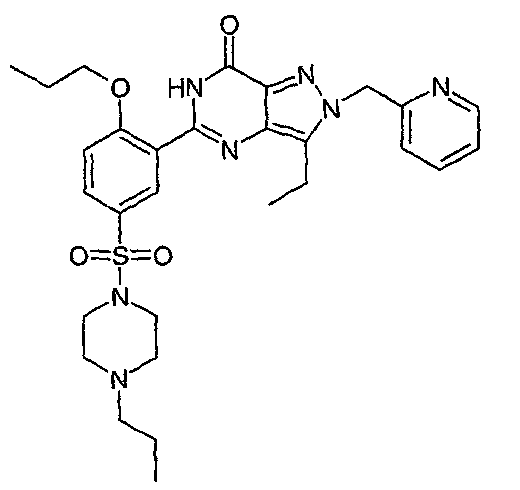

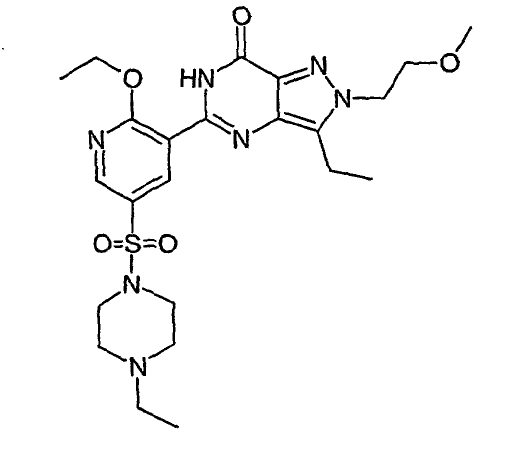

11. A process as claimed in any one of the preceding claims, wherein the group R^{x} of the compound of formula II represents -NH₂, -NHR^{a}, -N(R^{b})R^{c}, -SR^{d}, -SH, -OR^{e} (in which groups R^{a} to R^{e} each independently represent the same groups that R¹ as defined in Claim 1 may represent, except that they do not represent H) or halo.

12. A process as claimed in Claim 11, wherein R^{x} represents -NHR^{a}, -N(R^{b})R^{c}, -SR^{d}, -SH or -OR^{e}_{.}

13. A process as claimed in Claim 12, wherein R^{x} represents ethoxy.

14. A process as claimed in any one of the preceding claims, wherein the reaction is carried out in the presence of a solvent system that includes an aromatic hydrocarbon, chlorobenzene or a solvent of formula R^{x}H, wherein R^{x} is as defined in any one of Claims 1 or 11 to 13.

15. A process as claimed in Claim 14, wherein the solvent is toluene, xylene, chlorobenzene or ethanol.

16. A process as claimed in Claim 14 or Claim 15, wherein the reaction is carried out at reflux temperature.

17. A process as claimed in any one of the preceding claims, wherein the compound of formula II is prepared by way of reaction of a compound of formula IV, 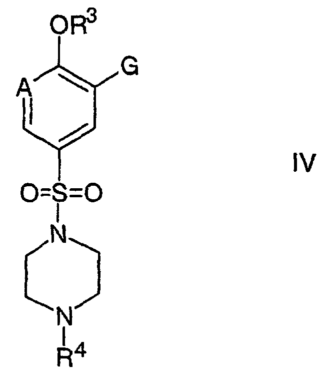 wherein G represents a carboxylic acid group (-C(O)OH) or a derivative thereof, and A, R³ and R⁴ are as defined in any one of Claims 1 and 6 to 10 (as appropriate), with an appropriate reagent for converting the group G to a -C(R^{x})=NH group, wherein R^{x} is as defined in any one of Claims 1 or 11 to 13.

18. A process as claimed in Claim 17, wherein, in the compound of formula IV, the group G represents -CN, -C(OR^{e})₃, -C(O)NH₂ or -C(=NOR^{f})NR^{e}₂, wherein R^{f} represents H or C₁₋₆ alkyl and R^{e} is as defined in Claim 11.

19. A process as claimed in Claim 18, wherein, when R^{x} represents -OR^{e} (wherein R^{e} represents C₁₋₆ alkyl (optionally interrupted by O), alkylHet or alkylaryl):
(a) a corresponding compound of formula IV in which G represents -CN is reacted with an alcohol of formula VA,
R_{α}OH VA
wherein R_{α} represents C₁₋₆ alkyl (optionally interrupted by O), alkylHet or alkylaryl, and Het is as defined in Claim 1, in the presence of a protic acid;
(b) a corresponding compound of formula IV in which G represents -C(O)NH₂ is reacted with an appropriate alkylating agent of formula VB,
R_{α}-Z¹ VB
wherein Z¹ represents a leaving group and R_{α} is as defined above; or
(c) a corresponding compound of formula IV in which G represents -C(OR_{α})₃, wherein R_{α} is as defined above, is reacted with ammonia, or an *N*-protected derivative thereof.

20. A process as claimed in Claim 18, wherein, when R^{x} represents -OR^{e} (wherein R^{e} represents Het or aryl), a corresponding compound of formula IV in which G represents -CN is reacted with a compound of formula VC,
R_{β}OH VC
wherein R_{β} represents Het or aryl, and Het is as defined in Claim 1.

21. A process as claimed in Claim 18, wherein, when R^{x} represents -NH₂:
(a) a corresponding compound of formula IV in which G represents -CN is reacted with hydrazine, hydroxylamine or *O*-lower alhyl hydroxylamine, followed by reduction of the resultant intermediate under standard conditions; or
(b) a corresponding compound of formula IV in which G represents -C(=NOR^{f})NR₂, wherein R^{f} is as defined in Claim 18, is reduced under standard conditions.

22. A process as claimed in Claim 18, wherein, when R^{x} represents -NH₂, -NHR^{a} or -N(R^{b})R^{c}, a corresponding compound of formula IV in which G represents -CN is reacted with a compound of formula VD,
HN(R_{χ})(R_{δ}) VD
wherein R_{χ} and R_{δ} independently represent H or R^{a}, and R^{a} is as defined in Claim 11.

23. A process as claimed in Claim 18, wherein, when R^{x} represents -SH:
(a) a corresponding compound of formula IV in which G represents -CN is reacted with hydrogen sulfide; or
(b) a corresponding compound of formula IV in which G represents -C(O)NH₂ is reacted with a reagent that effects oxygen-sulfur exchange.

24. A process as claimed in Claim 18, wherein, when R^{x} represents -SR^{d}, a corresponding compound of formula IV in which G represents -CN is reacted with a compound of formula VE,
R^{d}SH VE
wherein R^{d} is as defined in Claim 11.

25. A process as claimed in Claim 18, wherein, when R^{x} represents halo, a corresponding compound of formula IV in which G represents -C(O)NH₂ is reacted with a halogenating agent.

26. A process as claimed in any one of Claims 1 to 16, wherein the compound of formula II is prepared by way of reaction of another compound of formula II with a reagent that will convert one R^{x} group to another, wherein R^{x} is as defined in any one of Claims 1 or 11 to 13.

27. A process as claimed in Claim 26, wherein, when R^{x} represents -OR^{e} (wherein R^{e} represents C₁₋₆ alkyl, alkylHet or alkylaryl), a corresponding compound of formula II in which R^{x} represents Cl is reacted with a compound of formula VA, as defined in Claim 19.

28. A process as claimed in Claim 26, wherein, when R^{x} represents -NH₂, -NHR^{a} or -N(R^{b})R^{c}, a corresponding compound of formula II in which R^{x} represents Cl, -SH, -SR^{d} or -OR^{e}, wherein R^{d} and R^{e} are as defined in Claim 11, is reacted with an appropriate compound of formula VD, as defined in Claim 22, or an acid addition salt thereof.

29. A process as claimed in Claim 26, wherein, when R^{x} represents -SR^{d}, a corresponding compound of formula IV in which R^{x} represents -SH is reacted with a compound of formula VF,
R^{d}-Z² VF
wherein Z² represents a leaving group and R^{d} is as defined in Claim 11.

30. A process as claimed in any one of Claims 17 to 25, wherein the compound of formula IV is prepared by reaction of a compound of formula VI, 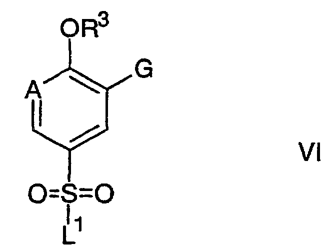 wherein L¹ is a leaving group and A, G and R³ are as defined in any one of Claims 1, 6, 7, 10, 17 and 18 (as appropriate), with a compound of formula VII, 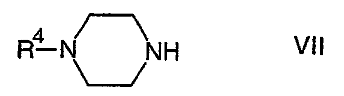 wherein R⁴ is as defined in any one of Claims 1 and 8 to 10.

31. A process as claimed in Claim 30, wherein the compound of formula VI is prepared by reaction of a compound of formula VIII, 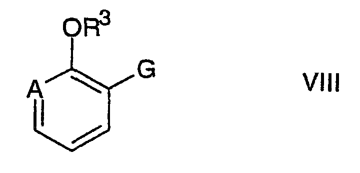 wherein A, G and R³ are as defined in any one of Claims 1, 6, 7, 10, 17 and 18 (as appropriate), with a reagent that may be used for the introduction of a -SO₂L¹ group into an aromatic or heteroaromatic ring system.

32. A process as claimed in any one of Claims 17 to 24, wherein the compound of formula IV is one in which G represents -CN or -C(O)NH₂, and is prepared by reaction of a compound of formula IX, 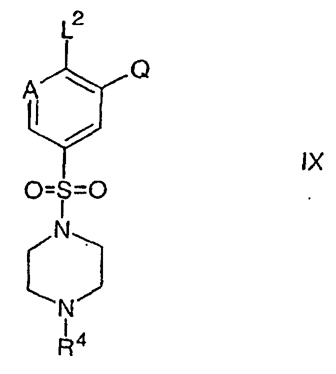 wherein Q represents -CN or -C(O)NH₂, L² represents a leaving group and A and R⁴ are as defined in any one of Claims 1 and 8 to 10, with a compound that will provide the group R³O.

33. A process as claimed in Claim 32, wherein the compound that will provide the group R³O is a C₁₋₆ alkyl alcohol.

34. A process as claimed in Claim 32 or 33, wherein the leaving group L² is chloro.

35. A process as claimed in any one of Claims 32 to 34, wherein the compound of formula IX is prepared by reaction of a compound of formula X, 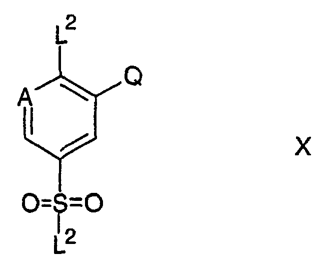 wherein Q and L² are as defined in Claim 32, and A is as defined in Claim 1, with a compound of formula VII as defined in Claim 30.

36. A process as claimed in any one of Claims 17 to 24, wherein the compound of formula IV is one in which G represents -CN, and is prepared by dehydration of a corresponding compound of formula IV in which G represents -C(O)NH₂.

37. A process as claimed in any one of Claims 17 to 19, 23 and 25, wherein the compound of formula IV in which G represents -C(O)NH₂ is prepared from a corresponding compound of formula IV in which G represents -C(O)OH by reaction with ammonia or a derivative thereof.

38. A compound of formula II, as defined in any one of Claims 1 and 11 to 13.

39. A compound according to Claim 38 wherein A represents -CH, R³ represents Et, R⁴ represents Me and R^{x} represents NH₂.

40. A compound according to Claim 38 wherein A represents -CH, R³ represents Et, R⁴ represents Et and R^{x} represents NH₂.

41. A compound of formula IV, as defined in Claim 18.

42. A compound according to Claim 39 wherein A represents N, R³ represents Et, R⁴ represents Et and G represents CO₂H.

43. A compound according to Claim 39 wherein A represents N, R³ represents Et, R⁴ represents Et and G represents CO₂Et.

44. A compound according to Claim 39 wherein A represents -CH, R³ represents Et, R⁴ represents Et and G represents CN.

45. A compound according to Claim 39 wherein A represents -CH, R³ represents Et, R⁴ represents Me and G represents CN.

## Patentansprüche

1. Verfahren zur Herstellung einer Verbindung der allgemeinen Formel I: 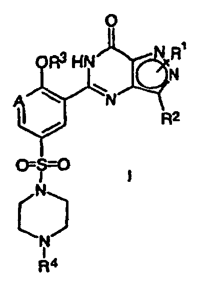 worin
A CH oder N wiedergibt;
R¹ H, Niederalkyl (wobei die Alkylgruppe gegebenenfalls durch O unterbrochen ist), Het, AlkylHet, Aryl oder Alkylaryl, wobei die letzteren fünf Gruppen alle gegebenenfalls mit einem oder mehreren Substituenten, ausgewählt aus Halogen, Cyano, Nitro, Niederalkyl, OR⁵, C(O)R⁶, C(O)OR⁷, C(O)NR⁸R⁹, NR^{10a}R^{10b} und SO₂NR^{11a}R¹¹b, substituiert sind (und/oder im Fall von Niederalkyl gegebenenfalls beendet sind), wiedergibt;
R² und R⁴ unabhängig Niederalkyl wiedergeben;
R³ Niederalkyl, wobei die Alkylgruppe gegebenenfalls durch Sauerstoff unterbrochen ist, wiedergibt;
Het eine gegebenenfalls substituierte vier- bis zwölfgliedrige, heterocyclische Gruppe, wobei die Gruppe ein oder mehrere Heteroatome, ausgewählt aus Stickstoff, Sauerstoff und Schwefel, enthält, wiedergibt; worin die Gruppen Het, die N- oder S-Atome enthalten, in ihrer N- oder S-oxidierten Form vorliegen können;
R⁵, R⁶, R⁷, R⁸, R⁹, R^{11a} und R^{11b} unabhängig H oder Niederalkyl wiedergeben;
R^{10a} und R^{10b} entweder unabhängig H oder Niederalkyl wiedergeben oder zusammen mit dem Stickstoffatom, an das sie gebunden sind, Azetidinyl, Pyrollidinyl oder Piperidinyl wiedergeben, wobei Niederalkyl C₁₋₆-Alkyl bedeutet, das wenn es eine ausreichende Anzahl an Kohlenstoffatomen gibt, linear oder verzweigt, gesättigt oder ungesättigt, cyclisch, acyclisch oder zum Teil cyclisch/acyclisch und/oder mit einem oder mehreren Halogenatomen substituiert sein kann,
wobei das Verfahren die Reaktion einer Verbindung von Formel II 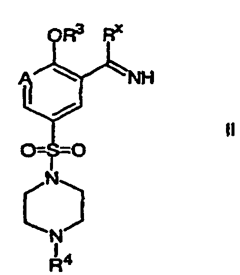 worin R^{x} eine durch ein Aminopyrazol substituierbare Gruppe darstellt, und A, R³ und R⁴ wie vorstehend definiert sind,
mit einer Verbindung der allgemeinen Formel III 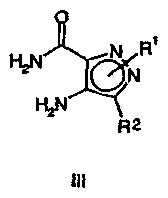 worin R¹ und R² wie vorstehend definiert sind, umfasst.

2. Verfahren nach Anspruch 1, wobei in der Verbindung der allgemeinen Formel I R¹ C₁₋₄-Alkyl wiedergibt, wobei die Alkylgruppe gegebenenfalls durch ein Sauerstoffatom unterbrochen ist und/oder gegebenenfalls durch eine Gruppe Het beendet ist.

3. Verfahren nach Anspruch 2, wobei R¹ lineares C₁₋₃-Alkyl wiedergibt, wobei die Alkylgruppe gegebenenfalls durch ein Sauerstoffatom unterbrochen ist, oder gegebenenfalls mit einer 2-Pyridinyl-Gruppe beendet ist.

4. Verfahren nach einem beliebigen der vorangehenden Ansprüche, wobei in der Verbindung der allgemeinen Formel IR² C₁₋₄-Alkyl wiedergibt.

5. Verfahren nach Anspruch 4, wobei R² lineares C₂₋₃-Alkyl wiedergibt.

6. Verfahren nach einem beliebigen der vorangehenden Ansprüche, wobei in der Verbindung der allgemeinen Formel I R³ C₁₋₅-Alkyl wiedergibt, wobei die Alkylgruppe gegebenenfalls durch ein Sauerstoffatom unterbrochen ist.

7. Verfahren nach Anspruch 6, wobei R³ lineares oder verzweigtes C₂₋₄-Alkyl wiedergibt, wobei die Alkylgruppe gegebenenfalls durch ein Sauerstoffatom unterbrochen ist.

8. Verfahren nach einem beliebigen der vorangehenden Ansprüche, wobei in der Verbindung der allgemeinen Formel I R⁴ C₁₋₃-Alkyl wiedergibt.

9. Verfahren nach Anspruch 8, wobei R⁴ C₁₋₂-Alkyl wiedergibt.

10. Verfahren nach einem beliebigen der vorangehenden Ansprüche, wobei die Verbindung aus Sildenafil oder einer beliebigen der nachstehenden vier Verbindungen ausgewählt ist,

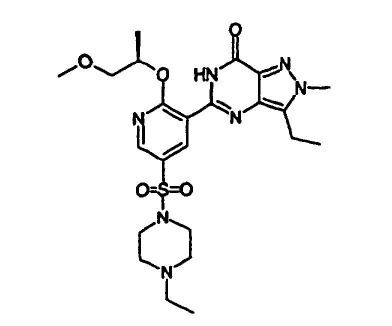

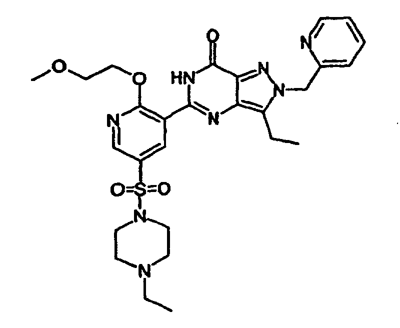

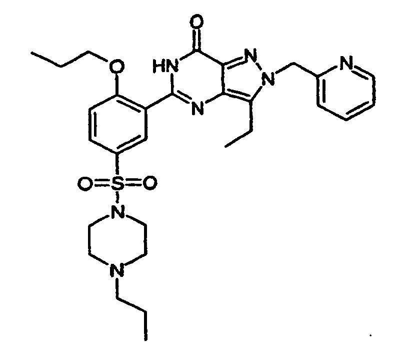

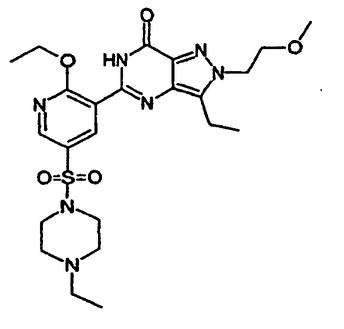

11. Verfahren nach einem beliebigen der vorangehenden Ansprüche, wobei die Gruppe R^{x} der Verbindung der Formel II -NH₂, -NHR^{a}, -N(R^{b})R^{c}, -SR^{d}, -SH, -OR^{e} (worin die Gruppe R^{a} bis R^{e} jeweils unabhängig die gleichen Gruppen wiedergeben, die R¹, wie in Anspruch 1 definiert, wiedergeben kann, mit der Ausnahme, dass sie kein H wiedergeben) oder Halogen wiedergibt.

12. Verfahren nach Anspruch 11, wobei R^{x} -NHR^{a}, -N(R^{b})R^{c}, -SR^{d}, -SH oder -OR^{e} wiedergibt.

13. Verfahren nach Anspruch 12, wobei R^{x} Ethoxy wiedergibt.

14. Verfahren nach einem beliebigen der vorangehenden Ansprüche, wobei die Reaktion in Gegenwart eines Lösungsmittel-Systems ausgeführt wird, das einen aromatischen Kohlenwasserstoff, Chlorbenzol oder ein Lösungsmittel der Formel R^{x}H einschließt, wobei R^{x} wie in einem beliebigen der Ansprüche 1 oder 11 bis 13 definiert ist.

15. Verfahren nach Anspruch 14, wobei das Lösungsmittel Toluol, Xylol, Chlorbenzol oder Ethanol darstellt.

16. Verfahren nach Anspruch 14 oder Anspruch 15, wobei die Reaktion bei der Rückflusstemperatur ausgeführt wird.

17. Verfahren nach einem beliebigen der vorangehenden Ansprüche, wobei die Verbindung der Formel II durch Reaktion einer Verbindung der Formel IV 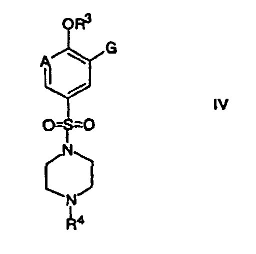 worin G eine Carbonsäure-Gruppe (-C(O)OH) oder ein Derivat davon wiedergibt und A, R³ und R⁴ wie in einem beliebigen der Ansprüche 1 und 6 bis 10 (falls geeignet) definiert sind, mit einem geeigneten Reagenz zum Umwandeln der Gruppe G zu einer Gruppe -C(R^{x})=NH, worin R^{x} wie in einem beliebigen der Ansprüche 1 oder 11 bis 13 definiert ist, hergestellt wird.

18. Verfahren nach Anspruch 17, wobei in der Verbindung der Formel IV die Gruppe G -CN, -C (OR^{e})₃, -C (O) NH₂ oder -C(=NOR^{f})NR^{e}₂ wiedergibt, worin R^{f} H oder C₁₋₆-Alkyl wiedergibt und R^{e} wie in Anspruch 11 definiert ist.

19. Verfahren nach Anspruch 18, wobei, wenn R^{x} -OR^{e} (worin R^{e} C₁₋₆-Alkyl (gegebenenfalls unterbrochen durch O), AlkylHet oder Alkylaryl wiedergibt) wiedergibt:
(a) eine entsprechende Verbindung der Formel IV, worin G -CN wiedergibt, mit einem Alkohol der Formel VA
R_{α}OH VA
worin R_{α} C₁₋₆-Alkyl (gegebenenfalls unterbrochen durch O), AlkylHet oder Alkylaryl wiedergibt und Het wie in Anspruch 1 definiert ist, in Gegenwart einer protischen Säure umgesetzt wird;
(b) eine entsprechende Verbindung der Formel IV, worin G -C(O)NH₂ wiedergibt, mit einem geeigneten Alkylierungsmittel der Formel VB
R_{α}-Z¹ VB
worin Z¹ eine Abgangsgruppe wiedergibt und R_{α} wie vorstehend definiert ist, umgesetzt wird; oder
(c) eine entsprechende Verbindung der Formel IV, worin G -C(OR_{α})₃ wiedergibt, worin R_{α} wie vorstehend definiert ist, mit Ammoniak oder einem N-geschützten Derivat davon umgesetzt wird.

20. Verfahren nach Anspruch 18, wobei, wenn R^{x} -OR^{e} (worin R^{e} Het oder Aryl wiedergibt) wiedergibt, eine entsprechende Verbindung der Formel IV, worin G -CN wiedergibt, mit einer Verbindung der Formel VC
R_{β}OH VC
worin R_{β} Het oder Aryl wiedergibt und Het wie in Anspruch 1 definiert ist, umgesetzt wird.

21. Verfahren nach Anspruch 18, wobei, wenn R^{x} -NH₂ wiedergibt:
(a) eine entsprechende Verbindung der Formel IV, worin G -CN wiedergibt, mit Hydrazin, Hydroxylamin oder O-Niederalkylhydroxylamin umgesetzt wird, gefolgt von Reduktion des erhaltenen Zwischenprodukts unter Standard-Bedingungen; oder
(b) eine entsprechende Verbindung der Formel IV, worin G -C(=NOR^{f})NR₂ wiedergibt, worin R^{f} wie in Anspruch 18 definiert ist, unter Standard-Bedingungen reduziert wird.

22. Verfahren nach Anspruch 18, wobei, wenn R^{x} -NH₂, -NHR^{a} oder -N(R^{b})R^{c} wiedergibt, eine entsprechende Verbindung der Formel IV, worin G -CN wiedergibt, mit einer Verbindung der Formel VD
HN(R_{χ} (R_{δ}) VD
worin R_{χ} und R_{δ} unabhängig H oder R^{a} wiedergeben und R^{a} wie in Anspruch 11 definiert ist, umgesetzt wird.

23. Verfahren nach Anspruch 18, wobei, wenn R^{x} -SH wiedergibt:
(a) eine entsprechende Verbindung der Formel IV, worin G -CN wiedergibt, mit Schwefelwasserstoff umgesetzt wird; oder
(b) eine entsprechende Verbindung der Formel IV, worin G -C(O)NH₂ wiedergibt, mit einem Reagenz umgesetzt wird, das Sauerstoff-Schwefel-Austausch bewirkt.

24. Verfahren nach Anspruch 18, wobei, wenn R^{x} -SR^{d} wiedergibt, eine entsprechende Verbindung der Formel IV, worin G -CN wiedergibt, mit einer Verbindung der Formel VE,
R^{d}SH VE
worin R^{d} wie in Anspruch 11 definiert ist, umgesetzt wird.

25. Verfahren nach Anspruch 18, wobei, wenn R^{x} Halogen wiedergibt, eine entsprechende Verbindung der Formel IV, worin G -C(O)NH₂ wiedergibt, mit einem Halogenierungsmittel umgesetzt wird.

26. Verfahren nach einem beliebigen der Ansprüche 1 bis 16, wobei die Verbindung der Formel II durch Reaktion einer weiteren Verbindung der Formel II mit einem Reagenz, das eine Gruppe R^{x} zu einer anderen umwandelt, worin R^{x} wie in einem beliebigen der Ansprüche 1 oder 11-13 definiert ist, hergestellt wird.

27. Verfahren nach Anspruch 26, wobei, wenn R^{x} -OR^{e} (worin R^{e} C₁₋₆-Alkyl, AlkylHet oder Alkylaryl wiedergibt) wiedergibt, eine entsprechende Verbindung der Formel II, worin R^{x} Cl wiedergibt, mit einer Verbindung der Formel VA, wie in Anspruch 19 definiert, umgesetzt wird.

28. Verfahren nach Anspruch 26, wobei, wenn R^{x} -NH₂, -NHR^{a} oder -N(R^{b})R^{c} wiedergibt, eine entsprechende Verbindung der Formel II, worin R^{x} Cl-, -SH, -SR^{d} oder -OR^{e} wiedergibt, worin R^{d} und R^{e} wie in Anspruch 11 definiert sind, mit einer geeigneten Verbindung der Formel VD, wie in Anspruch 22 definiert, oder einem Säureadditionssalz davon umgesetzt wird.

29. Verfahren nach Anspruch 26, wobei, wenn R^{x} -SR^{d} wiedergibt, eine entsprechende Verbindung der Formel IV, worin R^{x}-SH wiedergibt, mit einer Verbindung der Formel VF,
R^{d}-Z² VF
worin Z² eine Abgangsgruppe wiedergibt und R^{d} wie in Anspruch 1 definiert ist, umgesetzt wird.

30. Verfahren nach einem beliebigen der Ansprüche 17 bis 25, wobei die Verbindung der Formel IV durch Reaktion einer Verbindung der Formel VI 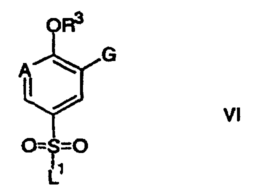 worin L¹ eine Abgangsgruppe darstellt, und A, G und R³ wie in einem beliebigen der Ansprüche 1, 6, 7, 10, 17 und 18 (falls geeignet) definiert sind, mit einer Verbindung der Formel VII 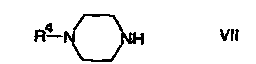 worin R⁴ wie in einem beliebigen der Ansprüche 1 und 8 bis 10 definiert ist, hergestellt wird.

31. Verfahren nach Anspruch 30, wobei die Verbindung der Formel VI durch Reaktion einer Verbindung der Formel VIII 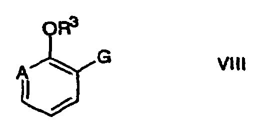 worin A, G und R³ wie in einem beliebigen der Ansprüche 1, 6, 7, 10, 17 und 18 (falls geeignet) definiert sind, mit einem Reagenz, das für die Einführung einer Gruppe -SO₂L¹ in ein aromatisches oder heteroaromatisches Ringsystem verwendet werden kann, hergestellt wird.

32. Verfahren nach einem beliebigen der Ansprüche 17 bis 24, wobei die Verbindung der Formel IV jene ist, worin G -CN oder -C(O)NH₂ wiedergibt, und durch Reaktion einer Verbindung der Formel IX 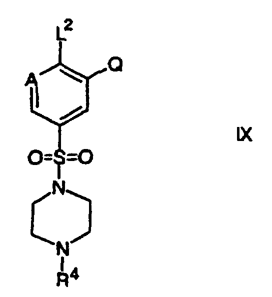 worin Q, -CN oder -C(O)NH₂ wiedergibt, L² eine Abgangsgruppe wiedergibt und A und R⁴ wie in einem beliebigen der Ansprüche 1 und 8 bis 10 definiert sind, mit einer Verbindung, die die Gruppe R³O bereitstellen wird, hergestellt wird.

33. Verfahren nach Anspruch 32, wobei die Verbindung, die die Gruppe R³O bereitstellen wird, einen C₁₋₆-Alkylalkohol darstellt.

34. Verfahren nach Anspruch 32 oder 33, wobei die Abgangsgruppe L² Chlor darstellt.

35. Verfahren nach einem beliebigen der Ansprüche 32 bis 34, wobei die Verbindung der Formel IX durch Reaktion einer Verbindung der Formel X 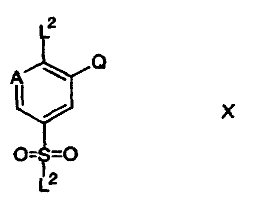 worin Q und L² wie in Anspruch 32 definiert sind, und A wie in Anspruch 1 definiert ist, mit einer Verbindung der Formel VII, wie in Anspruch 30 definiert, hergestellt wird.

36. Verfahren nach einem beliebigen der Ansprüche 17 bis 24, wobei die Verbindung der Formel IV jene ist, worin G -CN wiedergibt und durch Dehydratisierung der entsprechenden Verbindung der Formel IV, worin G -C(O)NH₂ wiedergibt, hergestellt wird.

37. Verfahren nach einem beliebigen der Ansprüche 17 bis 19, 23 und 25, wobei die Verbindung der Formel IV, worin G -C(O)NH₂ wiedergibt, aus einer entsprechenden Verbindung der Formel IV, worin G -C(O)OH wiedergibt, durch Reaktion mit Ammoniak oder einem Derivat davon hergestellt wird.

38. Verbindung der Formel II, wie in einem beliebigen der Ansprüche 1 und 11 bis 13 definiert.

39. Verbindung nach Anspruch 38, worin A -CH wiedergibt, R³ Et wiedergibt, R⁴ Me wiedergibt und R^{x} NH₂ wiedergibt.

40. Verbindung nach Anspruch 38, worin A -CH wiedergibt, R³ Et wiedergibt, R⁴ Et wiedergibt und R^{x} NH₂ wiedergibt.

41. Verbindung der Formel IV, wie in Anspruch 18 definiert.

42. Verbindung nach Anspruch 39, worin A N wiedergibt, R³ Et wiedergibt, R⁴ Et wiedergibt und G CO₂H wiedergibt.

43. Verbindung nach Anspruch 39, worin A N wiedergibt, R³ Et wiedergibt, R⁴ Et wiedergibt und G CO₂Et wiedergibt.

44. Verbindung nach Anspruch 39, worin A -CH wiedergibt, R³ Et wiedergibt, R⁴ Et wiedergibt und G CN wiedergibt.

45. Verbindung nach Anspruch 39, worin A -CH wiedergibt, R³ Et wiedergibt, R⁴ Me wiedergibt und G CN wiedergibt.

## Revendications

1. Procédé pour la production d'un composé de formule générale 1 : 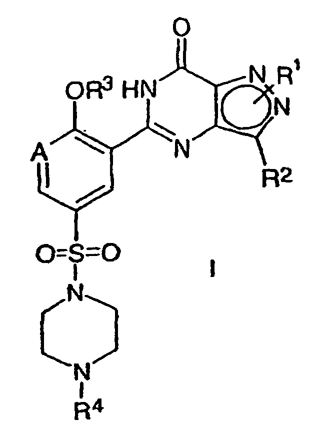 dans laquelle
A représente un groupe CH ou N ;
R¹ représente H, un groupe alkyle inférieur (groupe alkyle qui est facultativement interrompu par un atome de O), Het, alkylHet, aryle ou alkylaryle, ces cinq derniers groupes étant tous facultativement substitués (et/ou, dans le cas de groupes alkyle inférieurs, facultativement terminés) par un ou plusieurs substituants choisis entre des substituants halogéno, cyano, nitro, alkyle inférieur, OR⁵, C(O)R⁶, C(O)OR⁷, C(O)NR⁸R⁹, NR^{10a}R^{10b} et SO₂NR^{11a}R^{11b} _{;}
R² et R⁴ représentent indépendamment un groupe alkyle inférieur ;
R³ représente un groupe alkyle inférieur, groupe alkyle qui est facultativement interrompu par un atome d'oxygène ;
Het représente un groupe hétérocyclique tétra- à dodécagonal, facultativement substitué, qui contient un ou plusieurs hétéroatomes choisis entre des atomes d'azote, d'oxygène et de soufre, les groupes Het contenant des atomes de N ou S pouvant être présents sous la forme oxydée de N- ou S- ;
R⁵, R⁶, R⁷, R⁸, R⁹, R^{11a} et R^{11b} représentent indépendamment H ou un groupe alkyle inférieur ;
R^{10a} et R^{10b} représentent indépendamment H ou un groupe alkyle inférieur ou bien, conjointement avec l'atome d'azote auxquels ils sont fixés, représentent un groupe azétidinyle, pyrolidinyle ou pipéridinyle,
l'expression "alkyle inférieur" désignant un groupe alkyle en C₁ à C₆ qui, lorsqu'il existe un nombre suffisant d'atomes de carbone, peut être linéaire ou ramifié, saturé ou insaturé, cyclique, acyclique ou cyclique/acyclique mixte, et/ou substitué avec un ou plusieurs atomes d'halogènes,
procédé qui comprend la réaction d'un composé de formule II, 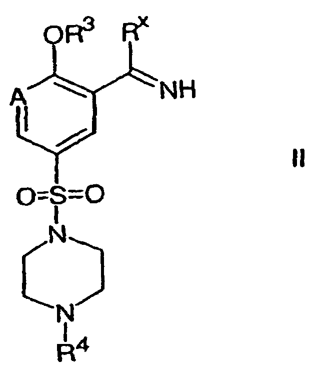 dans laquelle R^{x} représente un groupe pouvant être substitué avec un groupe aminopyrazole et A, R³ et R⁴ répondent aux définitions précitées,
avec un composé de formule générale III, 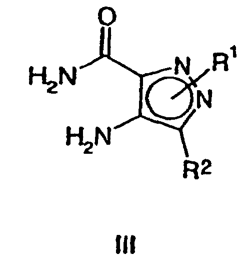 dans laquelle R¹ et R² répondent aux définitions précitées.

2. Procédé suivant la revendication 1, dans lequel, dans le composé de formule générale I, R¹ représente un groupe alkyle en C₁ à C₄, groupe alkyle qui est facultativement interrompu par un atome d'oxygène et/ou qui est facultativement terminé par un groupe Het.

3. Procédé suivant la revendication 2, dans lequel R¹ représente un groupe alkyle linéaire en C₁ à C₃, groupe alkyle qui est facultativement interrompu par un atome d'oxygène ou qui est facultativement terminé par un groupe 2-pyridinyle.

4. Procédé suivant l'une quelconque des revendications précédentes, dans lequel, dans le composé de formule générale I, R² représente un groupe alkyle en C₁ à C₄.

5. Procédé suivant la revendication 4, dans lequel R² représente un groupe alkyle linéaire en C₂ ou C₃.

6. Procédé suivant l'une quelconque des revendications précédentes, dans lequel, dans le composé de formule générale I, R³ représente un groupe alkyle en C₁ à C₅, groupe alkyle qui est facultativement interrompu par un atome d'oxygène.

7. Procédé suivant la revendication 6, dans lequel R³ représente un groupe alkyle linéaire ou ramifié en C₂ à C₄, groupe alkyle qui est facultativement interrompu par un atome d'oxygène.

8. Procédé suivant l'une quelconque des revendications précédentes, dans lequel, dans le composé de formule générale I, R⁴ représente un groupe alkyle en C₁ à C₃.

9. Procédé suivant la revendication 8, dans lequel R⁴ représente un groupe alkyle en C₁ ou C₂.

10. Procédé suivant l'une quelconque des revendications précédentes, dans lequel le composé est choisi entre le sildénafil et n'importe lequel des quatre composés suivants

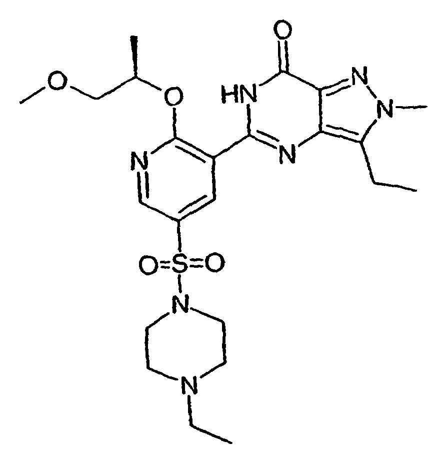

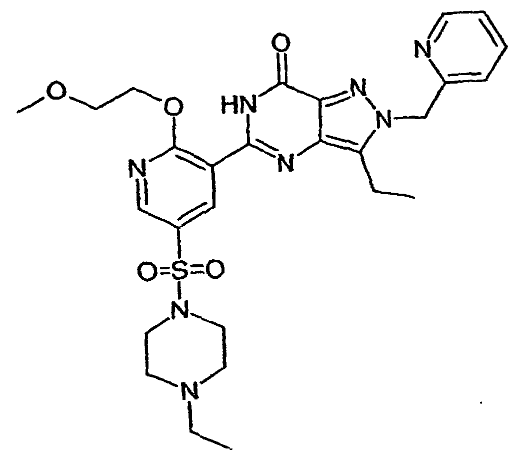

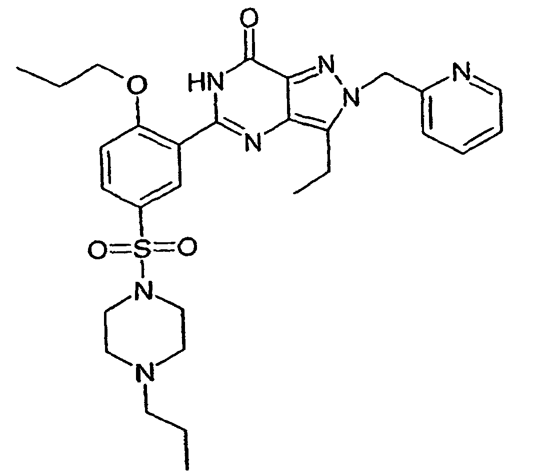

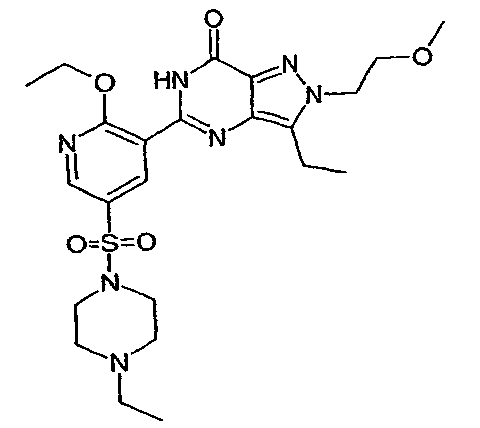

11. Procédé suivant l'une quelconque des revendications précédentes, dans lequel le groupe R^{x} du composé de formule II représente un groupe -NH₂, -NHR^{a}, -N(R^{b})R^{c}, -SR^{d}, -SH, -OR^{e} (dans lesquels les groupes R^{a} à R^{e} représentent chacun indépendamment les mêmes groupes que R¹ peut représenter, tel qu'il est défini dans la revendication 1, sauf qu'il ne représentent pas H) ou halogéno.

12. Procédé suivant la revendication 11, dans lequel R^{x} représente un groupe -NHR^{a}, -N(R^{b})R^{c}, -SR^{d}, -SH,ou -OR^{e}.

13. Procédé suivant la revendication 12, dans lequel R^{x} représente un groupe éthoxy.

14. Procédé suivant l'une quelconque des revendications précédentes, dans lequel la réaction est conduite en présence d'un système de solvant qui comprend un hydrocarbure aromatique, le chlorobenzène ou un solvant de formule R^{x}H, dans lequel R^{x} répond à la définition suivant l'une quelconque des revendications 1 ou 11 à 13.

15. Procédé suivant la revendication 14, dans lequel le solvant est le toluène, le xylène, le chlorobenzène ou l'éthanol.

16. Procédé suivant la revendication 14 ou la revendication 15, dans lequel la réaction est conduite à la température du reflux.

17. Procédé suivant l'une quelconque des revendications précédentes, dans lequel le composé de formule II est préparé par réaction d'un composé de formule IV, 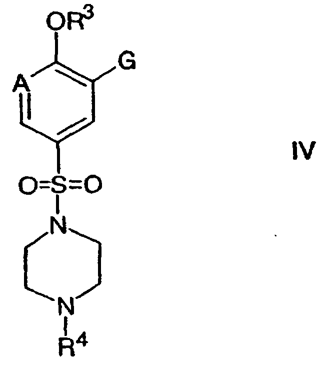 dans laquelle G représente un groupe acide carboxylique (-C(O)OH) ou un de ses dérivés, et A, R³ et R⁴ répondent aux définitions figurant dans l'une quelconque des revendications 1 et 6 à 10 (de la manière appropriée), avec un réactif approprié pour convertir le groupe G en un groupe -C(R^{x})=NH, dans lequel R^{x} répond à la définition figurant dans l'une quelconque des revendications 1 ou 11 à 13.

18. Procédé suivant la revendication 17, dans lequel, dans le composé de formule IV, le groupe G représente un groupe -CN, -C (OR^{e})₃, -C(O)NH₂ ou -C (=NOR^{f}) NR^{e}₂, dans lequel R^{f} représente H ou un groupe alkyle en C₁ à C₆ et R^{e} répond à la définition figurant dans la revendication 11.

19. Procédé suivant la revendication 18, dans lequel, lorsque R^{x} représente un groupe -OR^{e} (dans lequel R^{e} représente un groupe alkyle en C₁ à C₆ (facultativement interrompu par un atome de O), alkylHet ou alkylaryle) :
(a) un composé correspondant de formule IV dans laquelle G représente un groupe -CN est amené à réagir avec un alcool de formule VA,
RαOH VA
dans laquelle Rα représente un groupe alkyle en C₁ à C₆ (facultativement interrompu par un atome de O), alkylHet ou alkylaryle, et Het répond à la définition figurant dans la revendication 1, en présence d'un acide protique ;
(b) un composé correspondant de formule IV dans laquelle G représente un groupe -C(O)NH₂ est amené à réagir avec un agent alkylant approprié de formule VB,
Rα-Z¹ VB
dans laquelle Z¹ représente un groupe partant et Rα répond à la définition précitée ; ou
(c) un composé correspondant de formule IV dans laquelle G représente un groupe -C(ORα)₃, dans lequel Rα répond à la définition précitée, est amené à réagir avec l'ammoniac, ou un de ses dérivés *N*-protégés.

20. Procédé suivant la revendication 18, dans lequel, lorsque R^{x} représente un groupe -OR^{e} (dans lequel R^{e} représente un groupe Het ou aryle), un composé correspondant de formule IV dans lequel G représente un groupe -CN est amené à réagir avec un composé de formule VC,
RβOH VC
dans laquelle Rβ représente un groupe Het ou aryle et Het répond à la définition figurant dans la revendication 1.

21. Procédé suivant la revendication 18, dans lequel, lorsque R^{x} représente un groupe -NH_{2 :}
(a) un composé correspondant de formule IV dans laquelle G représente un groupe -CN est amené à réagir avec l'hydrazine, l'hydroxylamine ou une *O*(alkyle inférieur)hydroxylamine, avec ensuite une réduction de l'intermédiaire résultant dans des conditions classiques ; ou
(b) un composé correspondant de formule IV dans laquelle G représente un groupe -C(NOR^{f})NR₂, dans lequel R^{f} répond à la définition figurant dans la revendication 18, est réduit dans des conditions classiques.

22. Procédé suivant la revendication 18, dans lequel, lorsque R^{x} représente un groupe -NH₂, -NHR^{a} ou -N(R^{b})R^{c}, un composé correspondant de formule IV dans laquelle G représente un groupe -CN est amené à réagir avec un composé de formule VD,
HN(Rχ) (Rδ) VD
dans laquelle Rχ et Rδ représentent indépendamment H ou un groupe R^{a}, et R^{a} répond à la définition figurant dans la revendication 11.

23. Procédé suivant la revendication 18, dans lequel, lorsque R^{x} représente un groupe -SH :
(a) un composé correspondant de formule IV dans laquelle G représente un groupe -CN est amené à réagir avec de l'hydrogène sulfuré ; ou
(b) un composé correspondant de formule IV dans laquelle G représente un groupe -C(O)NH₂ est amené à réagir avec un réactif qui effectue un échange oxygène-soufre.

24. Procédé suivant la revendication 18, dans lequel, lorsque R^{x} représente un groupe -SR^{d}, un composé correspondant de formule IV dans laquelle G représente un groupe -CN est amené à réagir avec un composé de formule VE,
R^{d}SH VE
dans laquelle R^{d} répond à la définition figurant dans la revendication 11.

25. Procédé suivant la revendication 18, dans lequel, lorsque R^{x} représente un groupe halogéno, un composé correspondant de formule IV dans laquelle G représente un groupe -C(O)NH₂ est amené à réagir avec un agent d'halogénation.

26. Procédé suivant l'une quelconque des revendications 1 à 16, dans lequel le composé de formule II est préparé par réaction d'un autre composé de formule II avec un réactif qui convertit un groupe R^{x} en un autre, le groupe R^{x} répondant à la définition figurant dans l'une quelconque des revendications 1 ou 11 à 13.

27. Procédé suivant la revendication 26, dans lequel, lorsque R^{x} représente un groupe -OR^{e} (dans lequel R^{e} représente un groupe alkyle en C₁ à C₆, alkylHet ou alkylaryle), un composé correspondant de formule II dans lequel R^{x} représente un groupe Cl est amené à réagir avec un composé de formule VA, répondant à la définition figurant dans la revendication 19.

28. Procédé suivant la revendication 26, dans lequel, lorsque R^{x} représente un groupe -NH₂, -NHR^{a} ou -N(R^{b})R^{c}, un composé correspondant de formule II dans laquelle R^{x} représente un groupe Cl, -SH, -SR^{d} ou -OR^{e}, dans lequel R^{d} et R^{e} répondent aux définitions figurant dans la revendication 11, est amené à réagir avec un composé approprié de formule VD, répondant à la définition figurant dans la revendication 22, ou un de ses sels d'addition d'acides.

29. Procédé suivant la revendication 26, dans lequel, lorsque R^{x} représente un groupe -SR^{d}, un composé correspondant de formule IV dans laquelle R^{x} représente un groupe -SH est amené à réagir avec un composé de formule VF,
R^{d}-Z² VF
dans laquelle Z² représente un groupe partant et R^{d} répond à la définition figurant dans la revendication 11.

30. Procédé suivant l'une quelconque des revendications 17 à 25, dans lequel le composé de formule IV est préparé par réaction d'un composé de formule VI, 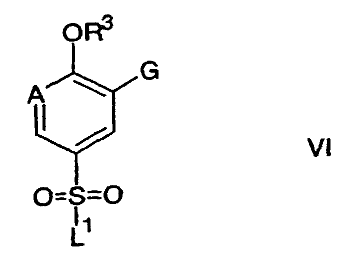 dans laquelle L¹ représente un groupe partant et A, G et R³ répondent aux définitions figurant dans l'une quelconque des revendications 1, 6, 7, 10, 17 et 18 (de la manière appropriée), avec un composé de formule VII, 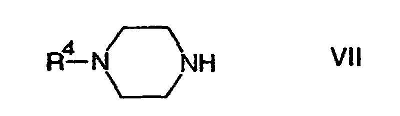 dans laquelle R⁴ répond à la définition figurant dans l'une quelconque des revendications 1 et 8 à 10.

31. Procédé suivant la revendication 30, dans lequel le composé de formule VI est préparé par réaction d'un composé de formule VIII, 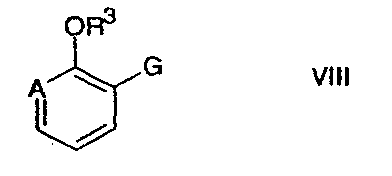 dans laquelle A, G et R³ répondent aux définitions figurant dans l'une quelconque des revendications 1, 6, 7, 10, 17 et 18 (de la manière appropriée), avec un réactif qui peut être utilisé pour l'introduction d'un groupe -SO₂L¹ dans un système de noyau aromatique ou hétéroaromatique.

32. Procédé suivant l'une quelconque des revendications 17 à 24, dans lequel le composé de formule IV est un composé dans lequel G représente un groupe -CN ou -C(O)NH₂ et est préparé par réaction d'un composé de formule IX, 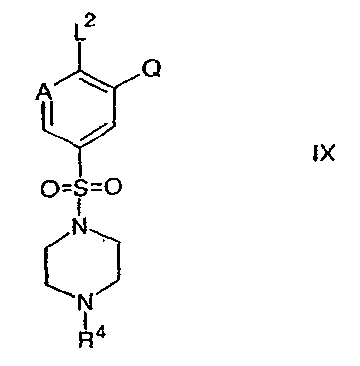 dans laquelle Q représente un groupe -CN ou -C(O)NH₂, L² représente un groupe partant et A et R⁴ répondent aux définitions figurant dans l'une quelconque des revendications 1 et 8 à 10, avec un composé qui fournira le groupe R³O.

33. Procédé suivant la revendication 32, dans lequel le composé qui fournira le groupe R³O est un alcool alkylique en C₁ à C₆.

34. Procédé suivant la revendication 32 ou 33, dans lequel le groupe partant L² est un groupe chloro.

35. Procédé suivant l'une quelconque des revendications 32 à 34, dans lequel le composé de formule IX est préparé par réaction d'un composé de formule X, 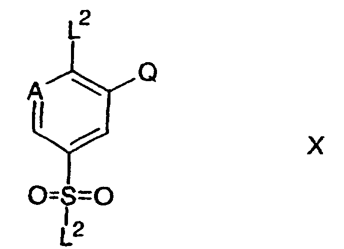 dans laquelle Q et L² répondent aux définitions figurant dans la revendication 32, et A répond à la définition figurant dans la revendication 1, avec un composé de formule VII répondant à la définition figurant dans la revendication 30.

36. Procédé suivant l'une quelconque des revendications 17 à 24, dans lequel le composé de formule IV est un composé dans lequel G représente un groupe -CN, et est préparé par déshydratation d'un composé correspondant de formule IV dans laquelle G représente un groupe -C(O)NH₂ .

37. Procédé suivant l'une quelconque des revendications 17 à 19, 23 et 25, dans lequel le composé de formule IV dans laquelle G représente un groupe -C(O)NH₂ est préparé à partir d'un composé correspondant de formule IV dans lequel G représente un groupe -C(O)OH par réaction d'ammoniac ou un de ses dérivés.

38. Composé de formule II, répondant à la définition figurant dans l'une quelconque des revendications 1 et 11 à 13.

39. Composé suivant la revendication 38, dans lequel A représente un groupe -CH, R³ représente un groupe Et, R⁴ représente un groupe Me et R^{x} représente un groupe NH₂.

40. Composé suivant la revendication 38, dans lequel A représente un groupe -CH, R³ représente un groupe Et, R⁴ représente un groupe Et et R^{x} représente un groupe NH₂.

41. Composé de formule IV, répondant à la définition figurant dans la revendication 18.

42. Composé suivant la revendication 39, dans lequel A représente un atome de N, R³ représente un groupe Et, R⁴ représente un groupe Et, et G représente un groupe CO₂H.

43. Composé suivant la revendication 39, dans lequel A représente un atome de N, R³ représente un groupe Et, R⁴ représente un groupe Et et G représente un groupe CO₂Et.

44. Composé suivant la revendication 39, dans lequel A représente un groupe -CH, R³ représente un groupe Et, R⁴ représente un groupe Et et G représente un groupe CN.

45. Composé suivant la revendication 39, dans lequel A représente un groupe -CH, R³ représente un groupe Et, R⁴ représente un groupe Me et G représente un groupe CN.
